(19) Europäisches Patentamt
European Patent Office
Office européen des brevets

(11) **EP 3 077 525 B1**

(12) **EUROPEAN PATENT SPECIFICATION**

(45) Date of publication and mention of the grant of the patent:
**27.02.2019 Bulletin 2019/09**

(21) Application number: **14867372.6**

(22) Date of filing: **05.12.2014**

(51) Int Cl.:
*C12P 7/62* *(2006.01)*          *C07C 67/08* *(2006.01)*
*C07C 69/40* *(2006.01)*          *C07C 51/43* *(2006.01)*
*C07C 55/10* *(2006.01)*

(86) International application number:
**PCT/US2014/068823**

(87) International publication number:
**WO 2015/085185 (11.06.2015 Gazette 2015/23)**

(54) **A PROCESS FOR PREPARING SUCCINATE ESTER**

VERFAHREN ZUR HERSTELLUNG VON SUCCINATESTER

PROCÉDÉ DE PRÉPARATION D'ESTER DE SUCCINATE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.12.2013 US 201361912578 P**

(43) Date of publication of application:
**12.10.2016 Bulletin 2016/41**

(73) Proprietor: **PTTGC Innovation America Corporation**
**Woburn, MA 01801 (US)**

(72) Inventors:
• **TOSUKHOWONG, Thidarat**
**Billerica, MA 01821 (US)**
• **WANG, Bin**
**Billerica, MA 01821 (US)**
• **MISTRY, Manav**
**Somerville, MA 02144 (US)**
• **DASARI, Rajesh**
**Lincoln, MA 01773 (US)**
• **WILSON, Zachary**
**Quincy, MA 02170 (US)**

(74) Representative: **Müller-Boré & Partner**
**Patentanwälte PartG mbB**
**Friedenheimer Brücke 21**
**80639 München (DE)**

(56) References cited:

KR-A- 20090 066 958     US-B1- 6 265 190
US-B1- 6 265 190

• LUQUE ET AL: "Chemical transformations of succinic acid recovered from fermentation broths by a novel direct vacuum distillation-crystallisation method", GREEN CHEMISTRY, ROYAL SOCIETY OF CHEMISTRY, GB, vol. 11, no. 2, 1 January 2009 (2009-01-01), pages 193-200, XP008134856, ISSN: 1463-9262, DOI: 10.1039/B813409J
• LUQUE, RAFAEL ET AL.: 'Chemical transformations of succinic acid recovered from fermentation broths by a novel direct vacuum distillation- crystallisation method' GREEN CHEMISTRY vol. 11, no. 2, 2009, pages 193 - 200, XP008134856
• ORJUELA, ALVARO ET AL.: 'A novel process for recovery of fermentation- derived succinic acid: process design and economic analysis' BIORESOURCE TECHNOLOGY vol. 139, July 2013, pages 235 - 241, XP028329576
• DELHOMME, CLARA ET AL.: 'Esterification of bio-based succinic acid in biphasic systems: Comparison of chemical and biological catalysts' JOURNAL OF MOLECULAR CATALYSIS B: ENZYMATIC vol. 80, August 2012, pages 39 - 47, XP028520184
• DAI, JIAN-YING ET AL.: 'Salting-out Extraction of Bio-based Chemicals' 2013 AMERICAN INSTITUTE OF CHEMICAL ENGINEERS (ALCHE) ANNUAL MEETING 07 November 2013, XP008183794

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention is in the field of producing specialty and commodity organic chemicals using biocatalysts that have been modified to increase their ability to produce one or other chemicals using renewable carbon resources. The present invention is related to producing 1,4-butanediol, gamma-butyrolactone and tetrahydrofuran from a succinic acid salt obtained from renewable carbon resources through biological fermentation involving biocatalysts. More specifically, the present invention is related to the production of succinate ester from a succinic acid salt present in a fermentation broth.

1,4-butanediol (BDO), gamma-butyrolactone (GBL), tetrahydrofuran (THF), and crystalline succinic acid are useful industrial chemicals. BDO is currently used as an industrial solvent in the manufacture of plastics and polyesters. BDO is also a precursor to useful chemicals like GBL and THF. It is a protic polar solvent, which is miscible with water. The current global market for BDO is about 3 billion pounds per year, almost exclusively produced from petrochemical processes. GBL is used as a solvent to replace environmentally harmful chlorinated solvents. GBL is an intermediate product in the preparation of pyrrolidones used as a raw material in the manufacture of herbicides, rubber additives and pharmaceuticals. THF is an aprotic, miscible solvent used in organic chemistry. It is also widely used in the production of resins and polymers.

The typical process to produce BDO starts from petrochemical derived acetylene which is reacted with formaldehyde using Reppe chemistry. The resulting 1,4-butynediol is then hydrogenated to form BDO. The most economical route for BDO manufacturing uses butane as a raw material. In the first stage of this process, butane is oxidized to produce maleic anhydride which in turn is converted to BDO via the BP/Lurgi Geminox process or the Davy Technology Process. The BP/Lurgi Geminox process recovers maleic anhydride as maleic acid and performs liquid-phase hydrogenation to produce a mixture of BDO with THF and/or GBL. In the Davy Technology Process, maleic anhydride is esterified to dimethyl maleate, which is then vaporized and fed to a vapor-phase hydrogenation system to produce dimethyl succinate. Dimethyl succinate undergoes hydrogenolysis reaction to produce GBL and BDO, which can be further converted into THF. These products are separated by distillation and methanol is recycled back to the esterification reactor.

These conventional processes for producing BDO, GBL, and THF are not sustainable as the raw materials are derived from petroleum feedstock. One of the possible pathways to derive a bio-based BDO is to esterify bio-succinic acid to dialkyl succinate and hydrogenating resulting dialkyl succinate to produce BDO, GBL, and THF. The main obstacle for the use of bio-succinic acid in the BDO production is the cost of the manufacturing process. There is a need to develop a bio-BDO manufacturing process in a cost effective manner.

Recently, there have been a significant advances in the production of succinic acid via fermentation of renewable carbon sources using biocatalyst, such as *E. coli*, *Actinobacillus succinogens* and *Mannheimia succiniproducens.* Bio-succinic acid can be polymerized with BDO to form a biodegradable polybutylene succinate (PBS) polymer. Furthermore, bio-succinic acid in an aqueous fermentation broth can be esterified with alcohol to make dialkylsuccinate, which in turn can be subjected to vapor-phase hydrogenation reaction to yield BDO, GBL, and THF. The route from bio-succinic acid to BDO via an alkyl ester of succinic acid has a significant advantage in reducing the carbon dioxide ($CO_2$) footprint over the conventional petro-based process for BDO manufacturing. As shown in Eq. [1], the *E. coli* strain useful in succinic acid production directly consumes $CO_2$ in order to make succinic acid. The *E. coli* strain producing succinic acid through a fermentation process requires about 0.5 mole of $CO_2$ to make each mole of succinic acid.

$$7\, C_6H_{12}O_6\ (\text{dextrose}) + 6\, CO_2 \rightarrow \quad 12\, C_4H_6O_4\ (\text{succinic acid}) + 6\, H_2O \quad \text{Eq. [1]}$$

The succinic acid fermentation process involving bacterial biocatalysts is typically maintained at near neutral pH by adding base to the fermentor as succinic acid is being produced. As a result, the product at the end of fermentation is in the form of a succinate salt. Several methods have been proposed to convert succinate salt back to succinic acid. Similarly, several methods have been proposed for the esterification of the succinic acid recovered from fermentation broth as well as the conversion of succinic acid ester into BDO, GBL, and THF through hydrogenation reactions. The present invention provides an integrated process for recovering succinic acid with minimal impurities from a fermentation broth and alkylating it to produce succinate ester.

Numerous investigations and efforts on designing an adequate downstream process for the recovery of succinic acid and its salt have been conducted and reviewed. The core separation technologies of different downstream routes include lime precipitation, chromatography including simulated moving bed chromatography, electro dialysis (ED), reactive extraction, adsorption involving ion exchange resins or zeolites, crystallization, etc. Most of these approaches are principally functional in lab and even at pilot scale, but industrial success will depend on scalability, operability, robustness, yield and costs. The objective of the present invention is to provide a novel process for recovering succinate ester from fermentation broth containing ammonium succinate which is cost-effective when scaled to industrial level.

U.S. Patent No. 5,168,055 disclosed a process for recovering succinic acid from a fermentation broth containing a succinic acid salt. According to this process, sulfuric acid is added to the broth to produce succinic acid and calcium sulfate (gypsum). Calcium sulfate resulting from this acidification process has very little to no commercial value and typically ends up as a landfill waste stream from the process.

U.S. Patents Nos. 5,034,105 and 5,143,834 disclosed an electro dialysis (ED) process to split a succinic acid salt in a fermentation broth to yield a base and succinic acid. Such a process has an advantage as no sulfate by-product is generated. However, an electro dialysis membrane is often subject to fouling from various proteins, macromolecules and multivalent ions in the fermentation broth leading to a very high replacement cost.

International Patent Application Publication No. WO2011/160760 and United States Patent Application Publication No. US 2013/0096343 disclosed a process comprising an acidification step to produce a mixture of succinic acid and ammonium sulfate, followed by a simulated moving bed (SMB) chromatography step to separate succinic acid from ammonium sulfate. The succinic acid stream is evaporated and crystallized to produce pure succinic acid.

The reactive extraction method for recovering carboxylic acid from fermentation broth containing either carboxylic acid or carboxylic acid salt has been described in a number of U.S. Patents. One issue associated with reactive extraction process is the challenge faced in recovering solvents at industry scale.

More than 25 sorbents were tested for uptake of succinic acid from aqueous solutions by Davison et al (2004). The best resins have a capacity of about 0.06 g of succinic acid/g of resin at moderate concentrations (1-5 g/L) of succinic acid. 70% recovery was achieved using hot water regeneration and this regeneration process was not stable over 10 cycles in the column. Alternative regeneration schemes using acid and base will increase chemical consumptions and generate more waste. Resin based separation is also susceptible to fouling if fermentation broth is not intensively pretreated.

Crystallization is one of the oldest separation and purification techniques known to mankind. Crystallization could be regarded as not only the final purification step but also the first recovery step for the downstream separation of succinic acid. Due to its robustness, operability and cost effectiveness, crystallization and precipitation from solutions are responsible for 70% of all solid materials produced by the chemical industry.

WO2011/123269 discloses a process for making THF, GBL, and BDO from diammonium succinate salt present in a fermentation broth. The process involves boiling the fermentation broth at above atmospheric pressure and at temperature of between 100-300°C. The objective of this process was to form overhead product containing water and ammonia and a liquid bottom product containing succinic acid and at least 20 wt% of water to prevent formation of amide by-products. A polar high-boiling solvent may be used in this step. Next, the bottom product is cooled to form solid portion containing succinic acid. The solid is recovered and hydrogenated in the presence of hydrogenation catalyst to produce THF, GBL and BDO. Example 4 in this patent application starts with 80 gram of 36% diammonium succinate solution and 80 gram of triglyme. During the evaporation, an additional amount of 3300g of water has to be gradually fed to the distilling mixture in order to prevent formation of side products, such as succinamic acid and succinimide. A total of 3313g of distillate was taken in the end. Then the solution was cooled down to precipitate 7.1g of solid. The solid had to be recrystallized again by adding 7.1g of hot water and cooled down to produce 3.9g of succinic acid. The succinic acid yield toward the crystal was calculated to be only 17% and it still contains 0.099wt% of succinamic acid. This process has an economic disadvantage as so much water needs to be added to the reaction (3300g water for 80 g of feed in this case) only to be distilled off. If this process is scaled up to an industrial level, it will require large amount of thermal energy for evaporation.

U.S. Patent Nos. 5,958,744 and 6,265,190 disclose a method that requires concentration of disodium succinate containing fermentation broth to 30% w/w and adjusting pH to 1.5-1.8 by adding ammonium, $H_2SO_4$ and $NH_4SO_4$ to yield succinic acid and ammonium sulfate. At this pH range, the solubility of succinic acid is low causing it to precipitate out. The precipitated succinic acid is redissolved in methanol. To produce a pure succinic acid, the methanol is evaporated and the succinic acid is recrystallized out. Ammonium sulfate is insoluble in methanol. Methanol is added into ammonium sulfate crystallizer to help ammonium sulfate to crash out. The co-product ammonium sulfate can be cracked thermally, preferably at about 290-310°C into ammonia and ammonium bisulfate. The evaporated methanol and water vapor could be condensed and reused. The level of impurities such as sulfur in the succinic acid obtained according to this process is not known.

There is a need to produce succinic acid esters with minimal sulfur content in the range of 100 ppm, preferably less than 50 ppm and most preferably less than 10 ppm as the hydrogenation catalysts used in the conversion of succinate ester to its hydrogenated products such as BDO, GBL and THF are known to be sensitive to sulfur contamination.

The present invention provides a novel and simple method for recovering succinic acid from a fermentation broth containing a succinic acid salt through acidification and estrifying succinic acid to yield dialkyl succinate which in turn is subjected to hydrogenation reaction to produce BDO, GBL and THF. Thus the production of bio-based BDO, GBL, or THF via the method of this present invention will have low carbon footprint and will help expand the portfolio for value-added green chemicals.

This present invention provides a process for preparing succinate ester from a succinic acid salt present in a fermentation broth. The succinate ester produced according to the present invention is hydrogenated to produce products selected from a group consisting of 1,4-butanediol (BDO), tetrahydrofuran (THF) and gamma-butyrolactone (GBL).

Succinic acid salt suitable for the present invention is produced using bacterial and fungal biocatalysts including yeast. Biocatalysts for succinic acid production can utilize a variety of carbon sources including glucose, sucrose, glycerol and cellulosic hydrolysates. Succinic acid is accumulated in the fermentation broth as a salt having a counter ion selected from a group consisting of alkaline metal, alkaline earth metal, ammonium, or alkyl ammonium group. Succinic acid is recovered as a free acid by means of acidifying the clarified fermentation broth using stronger acids. According to the present invention, phosphoric acid is used to acidify the fermentation broth containing ammonium succinate leading to the release of succinic acid and ammonium phosphate.

Among various succinic acid salts that can be manufactured through biological process according to the present invention, ammonium succinate is preferentially used in the present invention. The processes according to the present invention enable maximum recovery of succinic acid from a fermentation broth containing ammonium succinate and its conversion into succinate ester in a cost-effective manner.

In one embodiment of the present invention, succinic acid ester is obtained using a process involving evaporation, filtration, acidification-associated double displacement reaction, controlled crystallization, esterification and fractional distillation steps. In the first step of this embodiment, acidification of the fermentation broth containing ammonium succinate is carried out with a strong acid such as sulfuric acid (not within the scope of the invention) or phosphoric acid. This acidification of fermentation broth containing ammonium succinate causes a double displacement reaction where the cations and anions exchange partners. Ammonium succinate reacts with strong acid in a double displacement reaction leading to the formation of free succinic acid and an ammonium salt such as ammonium sulfate or ammonium phosphate depending on the nature of the strong acid added to the fermentation broth. After the completion of double displacement reaction, the acidified fermentation broth is subjected to evaporation under vacuum at an elevated temperature and a controlled crystallization process at a lower temperature. The succinic acid crystal from this first crystallization step is dissolved in an alcohol and subjected to an esterification reaction to produce a succinic acid ester which is purified through fractional distillation.

In one aspect of the present invention, the acidified fermentation broth is cooled to crystallize the succinic acid. In another embodiment of the present invention, the fermentation broth is subjected to evaporation before subjecting it to acidification. In a preferred aspect of this invention, the fermentation broth is subjected to evaporation and filtered before acidification step. In yet another aspect of the present invention, the succinic acid obtained from first crystallization step is subjected to a recrystallization step to further improve the quality of the succinic acid crystals.

Irrespective of the process steps followed to achieve first crystallization, the acidified fermentation broth is filtered or centrifuged to separate succinic acid crystals from mother liquor at the end of first crystallization step. The mother liquor from the first crystallization step is subjected to another evaporation step at an elevated temperature under vacuum followed by a second crystallization step at lower temperature under vacuum leading to co-crystallization of ammonium salt and succinic acid resulting from double displacement reaction. The co-crystallized product from the second crystallization step is dissolved or washed using methanol to separate the ammonium salt from succinic acid. Ammonium salt is insoluble in methanol and is recovered as crystalline ammonium salt through centrifugation or filtration while succinic acid is readily dissolved in methanol. The methanol phase containing succinic acid is subjected to an esterification reaction, either separately or combined with succinic acid from first crystallization step to produce succinic acid ester which is further purified through fractional distillation.

In another embodiment of the present invention, fermentation broth containing succinic acid salt is concentrated through evaporation followed by a salting out step. During the salting out process, the concentrated succinic acid salt is mixed with an organic solvent, e.g. methanol, ethanol, etc., to recover succinic acid salt as a solid precipitate while most of the impurities in the fermentation broth are retained in the organic solvent-water phase. When methanol is used as an organic solvent, methanol is recovered from methanol-water phase by distillation for reuse and the residual succinic acid salt in the aqueous phase is recycled back to the salting out step to maximize succinic acid salt recovery. Succinic acid salt recovered as a solid precipitate is mixed with methanol and subjected to a double displacement reaction with a strong acid to produce free succinic acid and newly formed ammonium salt of strong acid as the products of a double displacement reaction.

In the above aspect of the present invention, including the salting out step, sulfuric acid is used in the double displacement reaction to yield succinic acid and ammonium sulfate.

Succinic acid is fully soluble in methanol while the ammonium salt resulting from double displacement reaction is insoluble in methanol and recovered as a solid precipitate. Since sulfuric acid is capable of acting as an esterification catalyst as well, the succinic acid formed as a result of double displacement reaction in the presence of methanol is esterified to certain extent resulting in a mixture of succinic acid, monomethyl succinate, and dimethyl succinate in the methanol phase. By means of providing suitable condition for esterification reaction, the free succinic acid resulting from double displacement reaction in methanol phase can be fully esterified and dimethyl ester is recovered from methanol phase through fractional distillation. The dimethyl ester can be converted into other desirable esters such as diethyl succinate ester, dipropyl succinate ester and dibutyl succinate ester through appropriate transesterification reaction.

The succinic acid ester obtained according to the present invention is suitable for catalytic vapor-phase hydrogenation procedure to yield hydrogenation products such as BDO, GBL, and THF. The succinate ester can also be hydrolyzed to yield highly pure succinic acid.

## BRIEF DESCRIPTION OF THE DRAWINGS

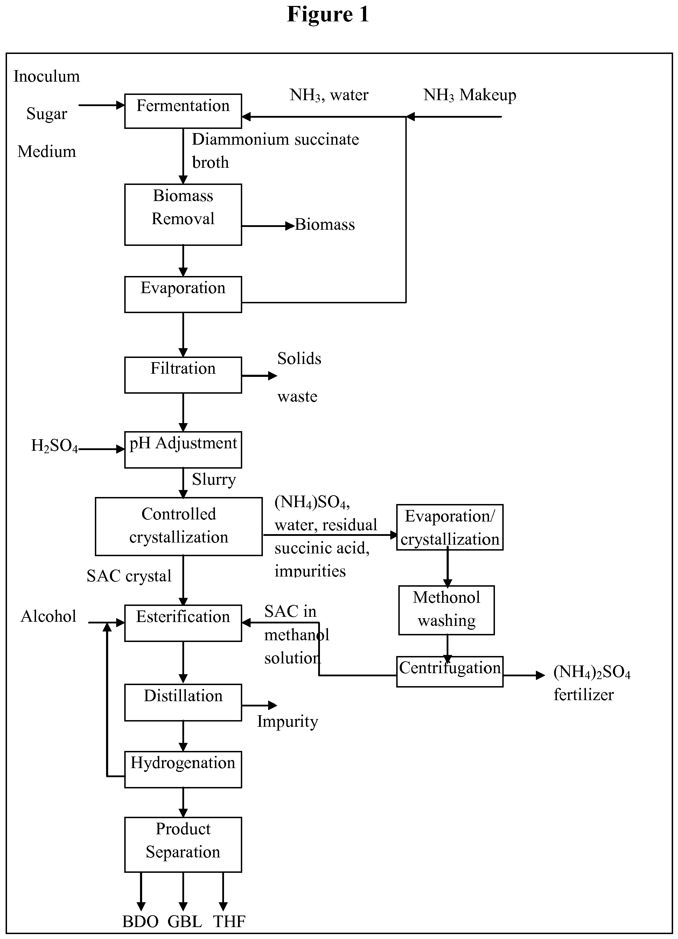

FIG. 1. Process flow diagram for the production of biomass-derived BDO, GBL and THF. In the fermentation process according to this flow diagram, succinic acid is produced as ammonium succinate. Acidification of fermentation broth containing ammonium succinate is achieved by the addition of sulfuric acid leading to the release of free succinic acid which is recovered.

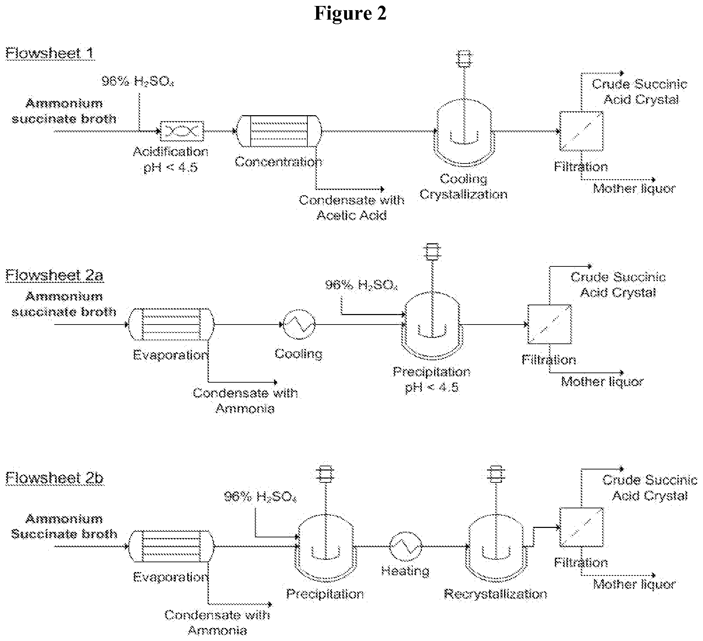

FIG. 2. Three flow sheet configurations for producing crude crystals of succinic acid. In the Flow sheet 1 configuration, fermentation broth containing ammonium succinate is acidified with the addition of sulfuric acid followed by concentration of fermentation broth by evaporation leading to the recovery of acetic acid as a condensate. The acidified, concentrated fermentation broth is cooled leading to the crystallization of succinic acid. The crystalline succinic acid is recovered through filtration. In the Flow sheet 2a configuration, fermentation broth containing ammonium succinate is concentrated by evaporation and ammonia is recovered in the condensate followed by cooling. Subsequently, the cooled concentrated fermentation broth is acidified and succinic acid is precipitated and recovered through filtration. In the Flow sheet 2b configuration, fermentation broth containing ammonium succinate is concentrated by evaporation and ammonia is recovered in the condensate. Sulfuric acid is used to acidify the concentrated fermentation broth and the succinic acid that precipitates out is heated and subjected to recrystallization to form larger and purer crystalline material. The acidification/precipitation, heating and crystallization steps can all take place in single equipment using a draft-tube baffled crystallizer connected to a recirculation loop where sulfuric acid can be added. Heat liberated from acidification, and optionally an auxiliary heater, can be used to redissolve fine particles. The residence time and the mixing in the draft-tube baffled crystallizer are to be controlled so that large crystals with high purity are obtained. In all three configurations, the succinic acid is recovered at the end through filtration or centrifugation as crude crystals. The mother liquor is subjected to further processing steps to recover remaining succinic acid.

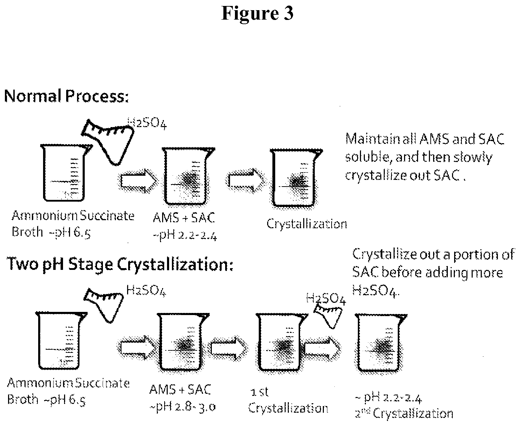

FIG. 3. One-stage and two-stage process for the acidification of fermentation broth containing ammonium succinate. Under the normal process for the acidification of fermentation broth containing ammonium succinate, also referred as one-stage process for acidification, sulfuric acid is added to the fermentation broth to bring down the pH of the fermentation broth from pH 6.5 to pH 2.2-2.4 in a single step. In the two-stage acidification process, initially the pH of the fermentation broth is lowered from pH 6.5 to an intermediate point (typically around pH 2.8-3.0) followed by a controlled crystallization step. Crystals formed by the first stage process are not harvested, but used as seeds to the second stage where the pH of the solution is further lowered to pH 2.2-2.5 and more succinic acid crystallizes out as the temperature is lowered. The acronyms "SAC" and "AMS" in this figure and thoughout this patent application represent succinic acid and ammonium sulfate, respectively.

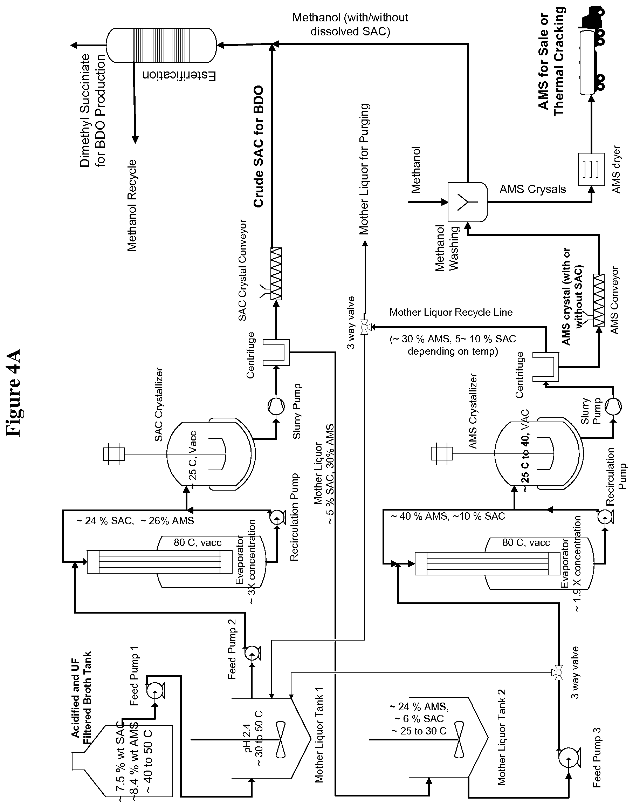

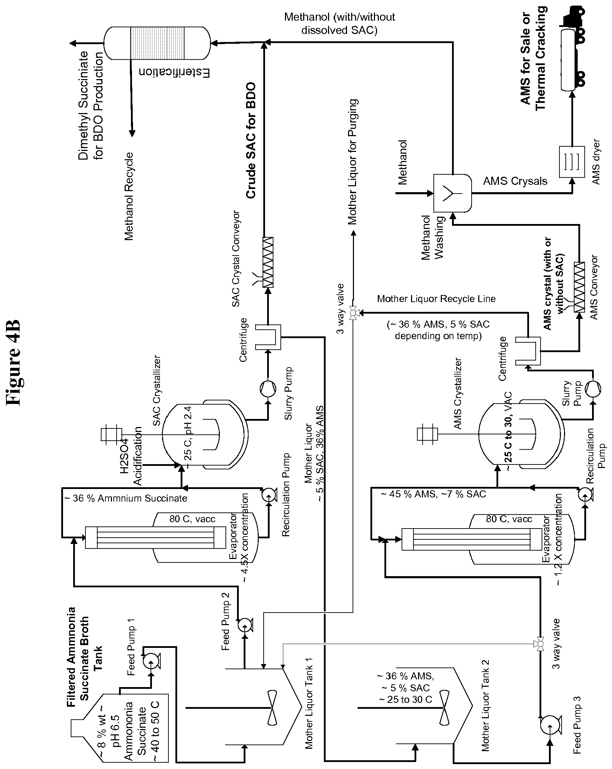

FIG. 4A and 4B. Detailed process flow diagram for producing dimethyl succinate involving evaporation, acidification, fractional crystallization, and esterification steps. The starting material for the production of dimethyl succinate according to this process flow diagram is fermentation broth containing ammonium succinate that has been clarified to remove biomass and protein. In Figure 4A, $H_2SO_4$ is used to acidify the fermentation broth leading to the protonation of succinic acid and formation of ammonium sulfate as a co-product. The acidified solution is concentrated in an evaporation step. Alternatively, as shown in Figure 4B, evaporation of clarified fermentation broth takes place prior to acidification with $H_2SO_4$. Subsequently in the first crystallization step, succinic acid, which has lower solubility than ammonium sulfate, is crystallized and separated from the mother liquor through centrifugation. The mother liquor, which is depleted in succinic acid, is further concentrated in an evaporator. In the second crystallization step, ammonium sulfate is crystallized with (or without) succinic acid. Ammonium sulfate is separated from the mother liquor by centrifugation. This mother liquor can be recycled to the first or the second evaporator. Succinic acid co-crystallized with the ammonium sulfate fraction is further separated from ammonium sulfate through a methanol washing step. Ammonium sulfate, which is insoluble in methanol can be centrifuged and recovered. Both the crude crystals of succinic acid obtained in the first crystallization step and the methanol wash stream containing succinic acid can be used as a feed to the esterification process. High-purity ammonium sulfate from the second crystallization step can be dried and sold as commercial fertilizer.

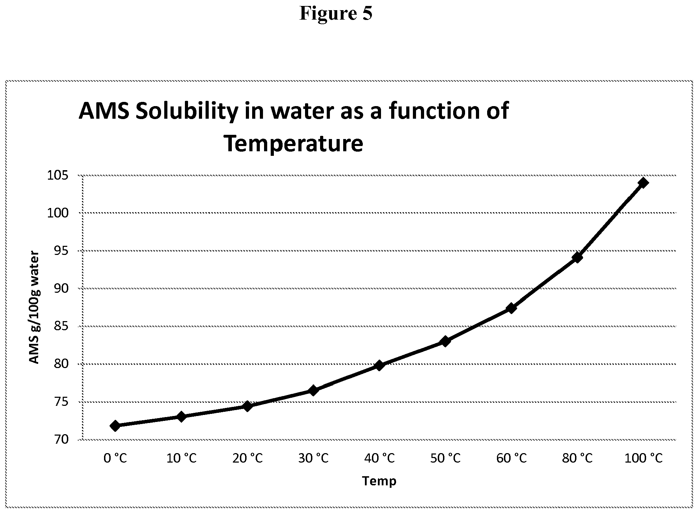

**FIG. 5.** Solubility curve for ammonium sulfate in water. There is an increase in the solubility of ammonium sulfate in water with the increase in temperature. Even at 0°C, about 70 grams of ammonium sulfate is still soluble in 100 grams of water.

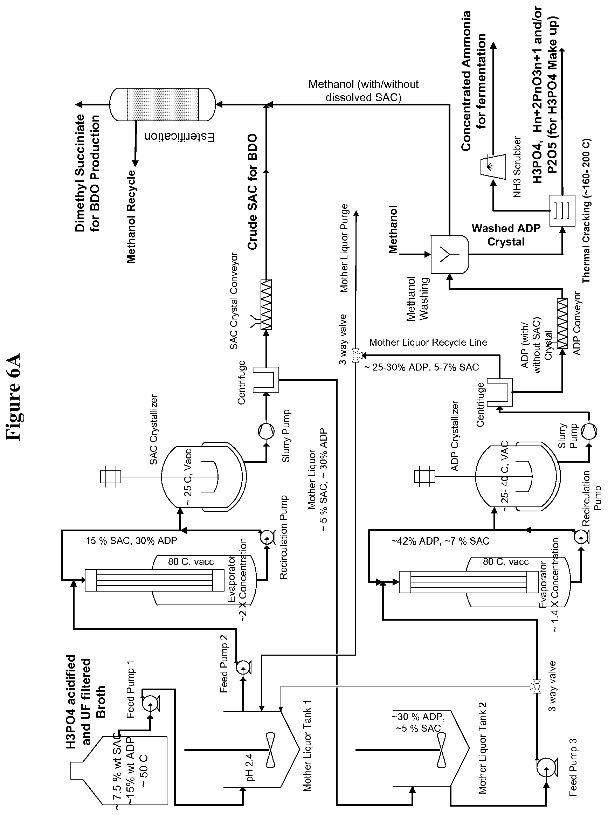

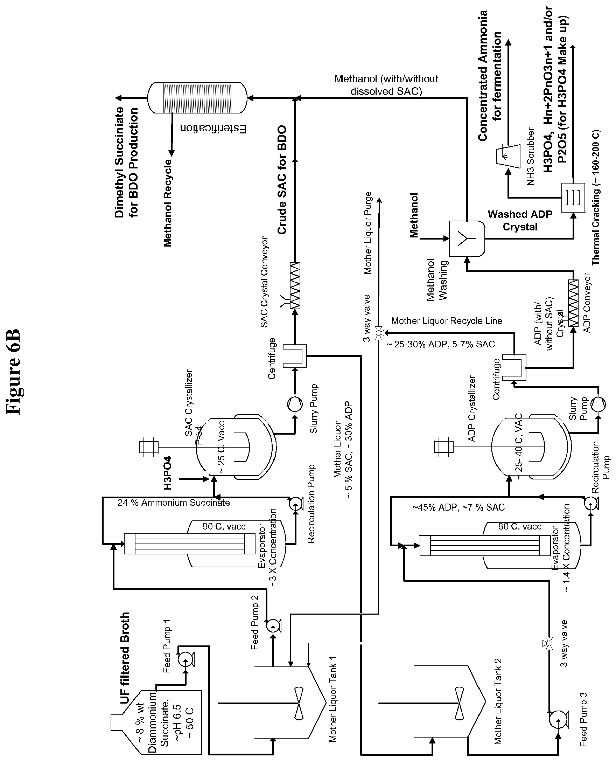

**FIG. 6A and 6B.** Detailed process flow diagram for producing dimethyl succinate involving evaporation, acidification, fractional crystallization, and esterification steps. The starting material for the production of dimethyl succinate according to this process flow diagram is fermentation broth containing ammonium succinate that has been clarified to remove biomass and protein. In Figure 6A, $H_3PO_4$ is used to acidify the fermentation broth leading to the protonation of succinic acid and formation of ammonium phosphate (ADP) as a co-product. The acidified solution is concentrated in an evaporation step. Alternatively, as shown in Figure 6B, evaporation of clarified fermentation broth takes place prior to acidification with phosphoric acid. Subsequently in the first crystallization step, succinic acid is crystallized and separated from the mother liquor through centrifugation. The mother liquor, which is depleted in succinic acid, is further concentrated in an evaporator. In the second crystallization step, ammonium phosphate is crystallized with (or without) succinic acid. Ammonium phosphate is separated from the mother liquor by centrifugation. This mother liquor can be recycled to the first or the second evaporator. Succinic acid co-crystallized with ammonium phosphate is further separated from ammonium phosphate through a methanol washing step. Ammonium phosphate, which is insoluble in methanol can be centrifuged and recovered. Both the crude crystals of succinic acid obtained in the first crystallization step and the methanol wash stream containing succinic acid can be used as a feed to the esterification process. High-purity ammonium phosphate from the second crystallization step can be thermally cracked to recover ammonia and phosphoric acid.

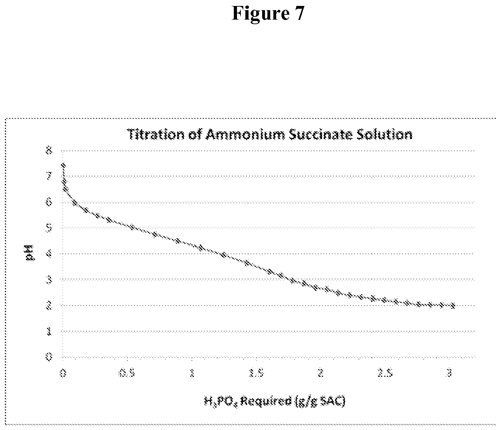

**FIG. 7.** Titration of phosphoric acid against ammonium succinate solution. To bring aqueous ammonium sulfate solution to pH 2.0, about 3 grams of phosphoric acid is required for every gram of ammonium succinate in the solution.

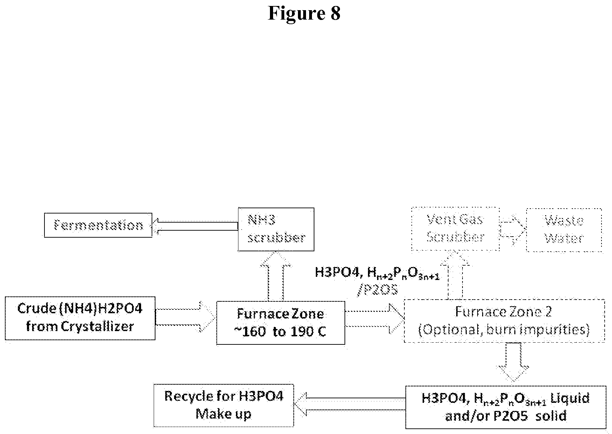

**FIG. 8.** Process for recycling ammonium phosphate. When phosphoric acid is used to acidify the fermentation broth containing ammonium succinate, ammonium phosphate is obtained as a byproduct. Shown in this process flow diagram are the steps for thermal conversion of crystalline ammonium phosphate to phosphoric acid, polyphosphoric acid and/or phosphorus pentoxide which can be recycled in the succinic acid recovery process according to the present invention.

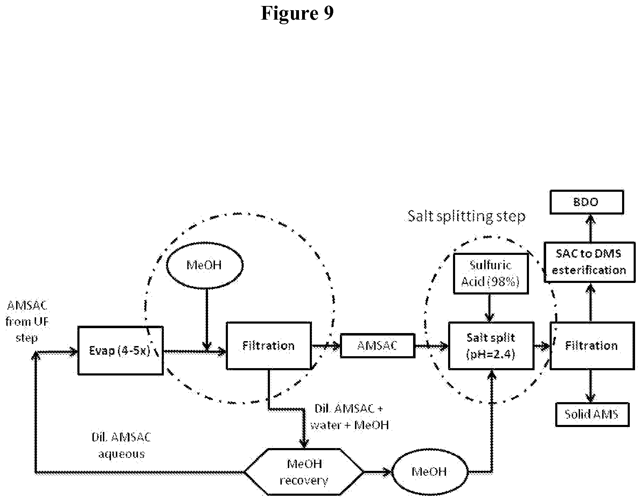

**FIG. 9.** Ammonium succinate salting out process for water removal from fermentation process. Fermentation broth containing ammonium succinate (AMSAC) is subjected to evaporation at an elevated temperature under vacuum and the concentrated fermentation broth is mixed with methanol to cause precipitation of ammonium succinate which is recovered through filtration. Methanol is recovered from methanol-water phase through distillation and recycled while the remaining dilute aqueous ammonium succinate solution is returned to evaporation step. The crystalline ammonium succinate is mixed with methanol in the presence of sulfuric acid to initiate a double displacement reaction accompanied by an esterification reaction. The insoluble ammonium sulfate resulting from double displacement reaction is recovered through filtration leaving behind a methanolic solution comprising succinic acid and a mixture of mono and dimethyl succinate. The methanolic solution is further reacted to completely convert succinic acid to dimethyl succinate, which is then recovered from methanol through fractional distillation.

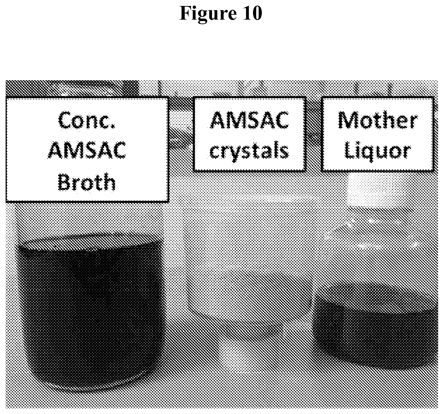

**FIG. 10.** Ammonium succinate (AMSAC) crystals and mother liquor derived from methanol treatment of fermentation broth containing ammonium succinate. The fermentation broth containing ammonium succinate is dark in color. With the addition of methanol to the fermentation broth, ammonium succinate is precipitated out as a white crystalline salt and filtered out from mother liquor. The mother liquor retains all the impurities in the original fermentation broth and appears as dark as the original fermentation broth.

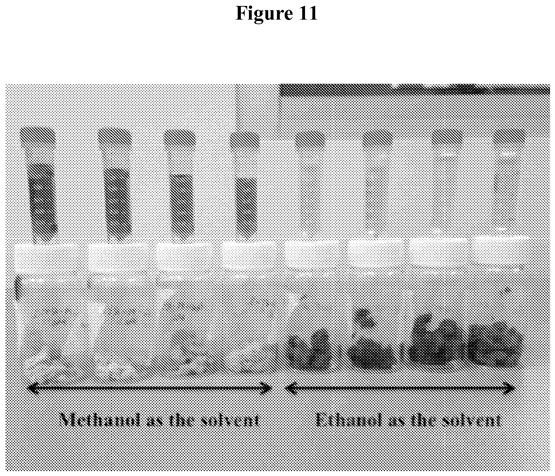

**FIG. 11.** Comparison of methanol and ethanol treatment of fermentation broth containing ammonium succinate. Methanol as a solvent specifically precipitates ammonium succinate as a white crystalline solid leaving behind all the impurities in the mother liquor. On the other hand with ethanol treatment, most of the impurities are precipitated along with ammonium succinate and the resulting ammonium succinate fraction appears dark and the mother liquor is light in color. Samples shown in the figure are numbered 1 through 8 from left to right. Samples 1 and 2 had methanol: concentrated broth ratio of 4 and samples 3 and 4 had methanol: concentrated broth ratio of 5. Samples 1 and 3 were treated with methanol at room temperature and samples 2 and 4 were treated with methanol at 60°C. Samples 5 and 6 had ethanol: concentrated broth ratio of 4 and samples 7 and 8 had ethanol: concentrated broth ratio of 5. Samples 5 and 7 were treated with ethanol at room temperature and samples 6 and 8 were treated with ethanol at 60°C.

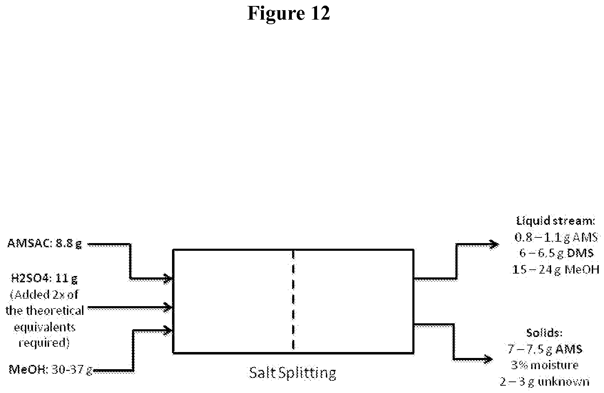

**FIG. 12.** Combined double displacement and esterification reactions involving solid ammonium succinate (AMSAC fraction obtained from fermentation broth containing ammonium succinate), methanol and sulfuric acid. The liquid fraction resulting from this combined double displacement and esterification reactions comprises succinic acid, monomethyl succinate and dimethyl succinate. The solid fraction resulting from this combined double displacement and esterification reactions comprises ammonium sulfate (AMS).

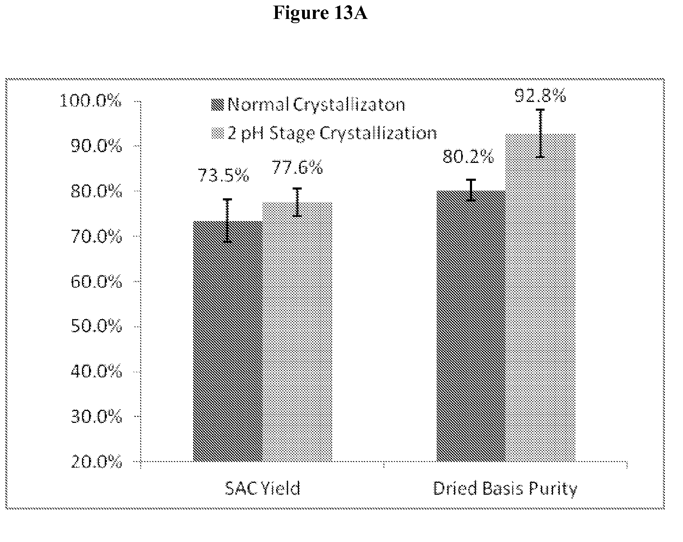

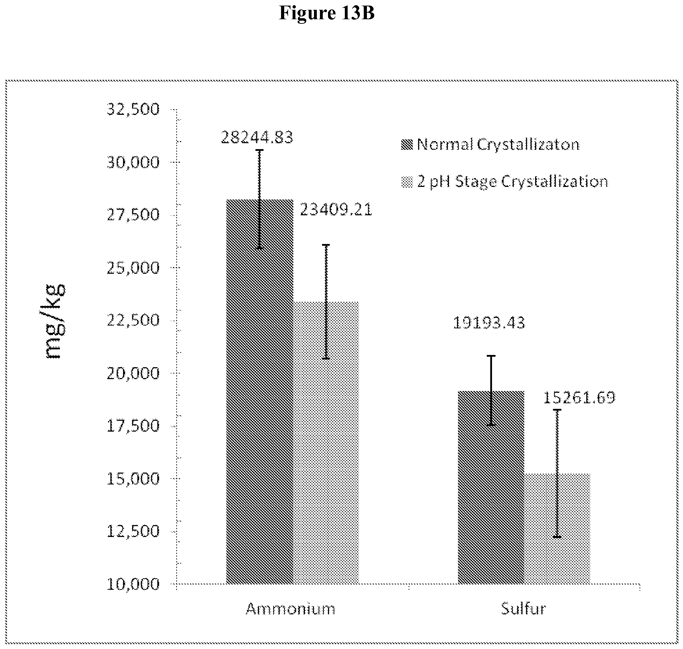

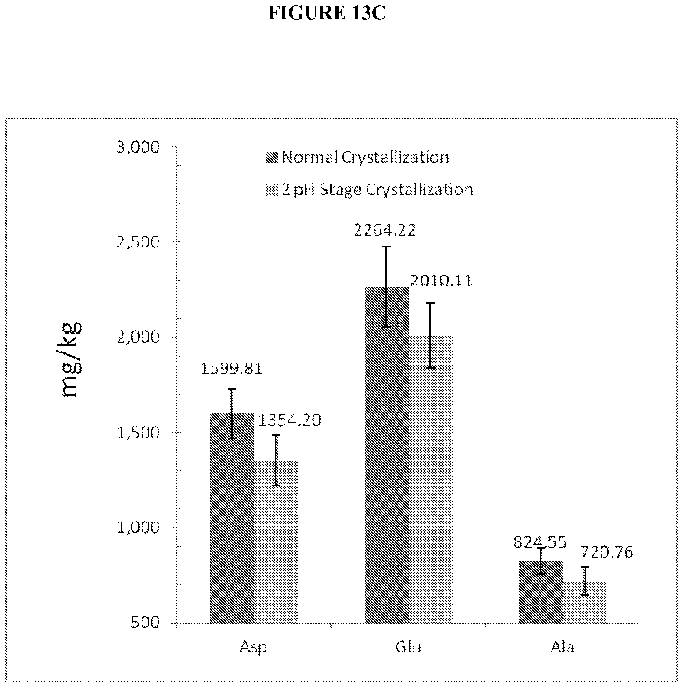

**FIG. 13A, 13B and 13C.** Comparison succinic acid crystals resulting from one-stage and two-stage acidification of fermentation broth containing ammonium succinate. Figure 13A provides the comparison of one-stage and two-stage acidification processes in terms of succinic acid yield and purity. While the yield of unwashed succinic acid crystals is comparable under both processes, the dried basis purity of succinic acid crystal is about 12% higher under two-stage acidification process as compared to the one-stage acidification process. Figure 13B provides the comparison of level of ammonium and sulfur impurities in the succinic acid crystals obtained through one-stage and two-stage acidification processes. Figure 13C provides the comparison of level of aspartic acid, glutamic acid and alanine impurities in the succinic acid crystals obtained through one-stage acidification process and two-stage acidification process. In the Figures 13A, 13B and 13C, the one-stage acidification process is referred as "Normal Crystallization" process.

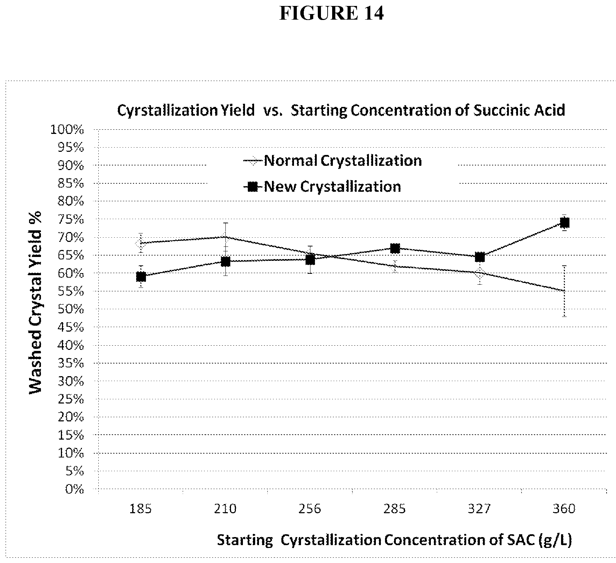

**FIG. 14.** Effect of succinic acid salt concentration on the yield of succinic acid crystal under one-stage acidification process and two-stage acidification process. The fermentation broth containing ammonium succinate was evaporated and subjected to ultrafiltration and the concentration of succinic acid salt was determined in the permeate from ultrafiltration step. In this Figure, the one-stage acidification process is referred as "Normal Crystallization".

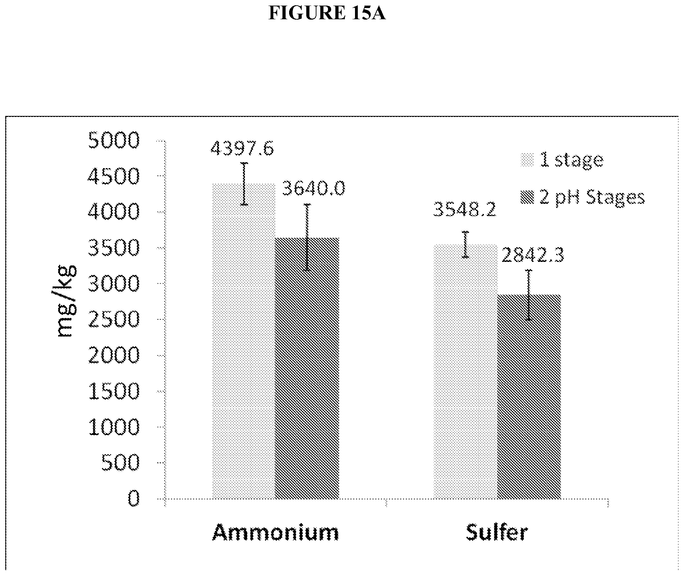

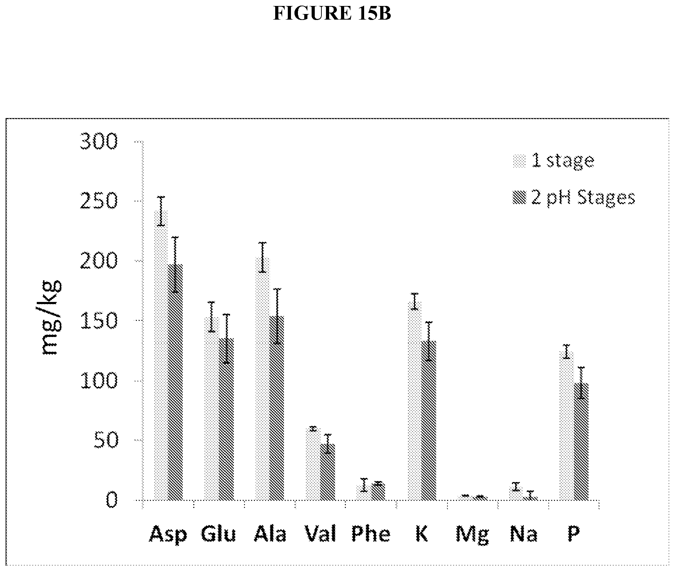

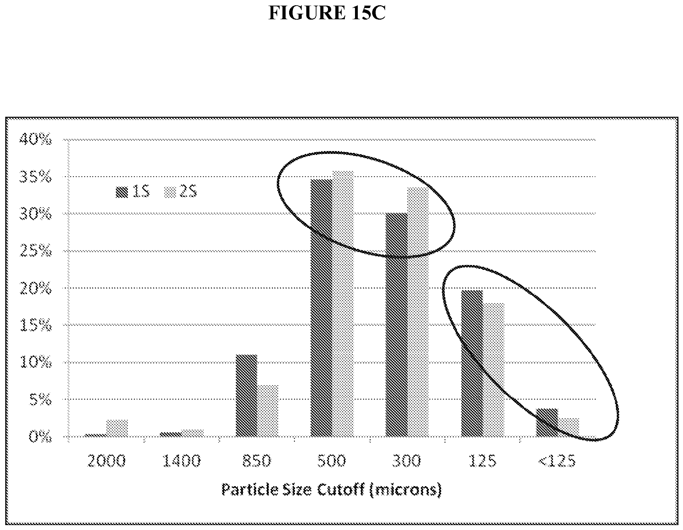

**FIG. 15.** Compositional and particle size analysis of succinic acid crystals obtained either through a one-stage acidification process or two-stage acidification process. Figure 15A provides the level of ammonium and sulfur impurities in the succinic acid crystals obtained from one-stage acidification process (1 stage) and two-stage acidification process (2pH stages). Figure 15B provides the level of various amino acid impurities in the succinic acid crystals obtained from one-stage acidification process (1 stage) and two-stage acidification process (2pH stages). Figure 15C provides the particle size distribution of succinic acid crystals obtained form one-stage acidification process (1S) and two-stage acidification process (2S).

As used in this invention, the term "bio-succinic acid" means succinic acid derived from renewable carbon sources through fermentation process involving biocatalysts. Succinic acid is accumulated in the fermentation broth as a succinic acid salt which is subjected to down-stream processing to recover succinic acid.

As used in this invention, the term "bio-BDO" means BDO derived from hydrogenation reaction involving bio-succinic acid as a starting material. In the first step of the process for producing bio-BDO, bio-succinic acid is esterified to produce bio-succinic acid ester which in turn is used as a substrate in the hydrogenation reaction to yield bio-BDO.

As used in this invention, the term "evaporation" means subjecting an aqueous solution to elevated temperatures to reduce the water content of the aqueous solution. The evaporation process is preferentially carried out under vacuum, As used in this invention, the term "concentration" means reducing the solvent content of an aqueous solution with reference to its solute content.

As used in this invention, the term "acidification" means adding an acid to an aqueous solution to reduce the pH of the said aqueous solution. The acidification of the fermentation broth containing a succinic acid salt in accordance with the present invention can be achieved either in a single step or in two steps. The acidification of the fermentation broth done in a single step is referred as "one-stage acidification" or "normal crystallization process". When the fermentation broth containing a succinic acid salt is acidified in two steps, it is referred as "two-stage acidification". In the two-stage acidification process, initially the pH of the fermentation broth is lowered from pH 6.5 to an intermediate point (typically around pH 2.8-3.0) followed by a controlled crystallization step. Crystals formed by the first stage process are not harvested, but used as seeds to the second stage where the pH of the solution is further lowered to pH 2.2-2.5 and more succinic acid crystallizes out as the temperature is lowered.

As used in this invention, the term "crystallization" means to precipitate dissolved solute in an aqueous solution by means of varying the temperature of the aqueous solution.

As used in this invention, the term "controlled crystallization" means to precipitate dissolved solute in an aqueous solution by means of varying the temperature of the aqueous solution in a pre-defined rate.

As used in this invention, the term "filtration" means removal of particulate matter in a solution by means of passing the solution through a coarse filter to retain the particulate matter. The filtration process is carried out either under gravitational flow or differential pressure across the membrane.

As used in this invention, the term "first crystallization" means the recovery of succinic acid from fermentation broth containing ammonium succinate following an acidification step. The acidification step involves the addition of an acid to the fermentation broth containing ammonium succinate to bring the pH of the fermentation broth to 2.4 or below.

As used in this invention, the term "second crystallization" means the recovery of ammonium sulfate or ammonium phosphate from the mother liquor resulting from the removal of succinic acid in the first crystallization step. To begin with a fermentation broth containing ammonium succinate is used. When sulfuric acid (not within the scope of the invention) is used in the acidification step, ammonium sulfate is recovered in the second crystallization step. Herein, as phosphoric acid is used in the acidification step, ammonium phosphate is recovered in the second crystallization step.

As used in this invention, the term "salting out" means precipitating ammonium succinate from a fermentation broth with dissolved ammonium succinate with the addition of methanol. The resulting ammonium succinate precipitate is recovered either through filtration or centrifugation.

As used in this invention, the term "salt splitting" means a double displacement reaction in which ammonium succinate is mixed with sulfuric acid in the presence of methanol. Presence of sulfuric acid causes the protonation of succinic acid from ammonium succinate accompanied by the formation of ammonium sulfate. Succinic acid resulting from double displacement reaction simultaneously enters into an esterification reaction with methanol in the presence of sulfuric acid as a catalyst leading to the accumulation of monomethyl succinate and dimethyl succinate. Depending on the rate of double displacement reaction and esterification reaction, varying amounts of succinic acid, ammonium sulfate, monomethyl succinate and dimethyl succinate accumulate in the reaction mixture at the end of the salt splitting reaction.

As used in this invention, the term "double displacement reaction" means a chemical reaction in which the anions and cations exchange partners. For example, in a double displacement reaction involving ammonium succinate and sulfuric acid, a proton from sulfuric acid replaces the ammonium cation in ammonium succinate leading to the formation of succinic acid. Simultaneously, ammonium cation reacts with sulfate anion of sulfuric acid to form ammonium sulfate.

As used in this invention, the term "quality of succinic acid crystals" means relative purity of the succinic acid crystals recovered using the process according to the present invention. For example, the objective of the present invention is to recover succinic acid crystals from a fermentation broth containing ammonium succinate with minimal sulfur content so that there is no interference in the functioning of the catalysts that are useful in the further downstream processing leading to the production of BDO, THF and GBL.

The present invention provides a method for producing BDO, GBL and THF from succinic acid derived from a fermentation process. Figure 1 provides a process flow diagram for the production of biomass-derived BDO, GBL, and THF. In a succinic acid producing fermentation suitable for the present invention, a variety of biocatalysts and a wide range of substrates suitable for microbial fermentation are used. The biocatalyst suitable for the present invention can be derived from a variety of microorganisms ranging from gram negative bacteria to fungi including yeast strains. The carbohydrate materials suitable for the present invention are glucose, sucrose, glycerol and lignocellulosic hydrolysates obtained from a variety of materials rich in six-carbon and five-carbon sugars. It is preferable to have the biocatalysts with the ability to use both six-carbon and five-carbon sugars simultaneously. One desirable characteristic of any biocatalyst suitable for the present invention is that biocatalysts show a high titer and high yield for succinic acid production. The terms "succinic acid yield" and "yield" refer to the mole of the succinic acid produced per mole of carbohydrate material consumed. The terms "succinic acid titer" and "titer" as used in the present invention refer to the amount of succinic acid produced per unit volume of the fermentation broth per unit period of time (g/L/hr). Another desirable feature of the biocatalysts suitable for the present invention is the ability to grow in a minimal mineral salt medium without the need for any additional nutrient sources such as yeast extract or corn steep liquor. The fermentation process can be carried out either under anaerobic condition or aerobic condition or microaerobic condition.

In the biological fermentation process to produce succinic acid, inorganic alkali and trace nutrient chemicals are added to the fermentor to maintain the condition where the organisms can function optimally. For example, *E. coli* strain KJ122 obtained through genetic manipulations produces succinic acid at the highest yield when the pH is around 6.5-7.0. With the production of succinic acid during the fermentation process, the pH of the fermentation medium decreases significantly. In order to maintain the pH of the fermentation medium, bases such as potassium hydroxide, sodium hydroxide, and ammonium hydroxide are added gradually during the course of the fermentation. As a result, at the end of the fermentation process, the succinic acid accumulates in the fermentation medium as succinic acid salt. For example when ammonium hydroxide is used as the neutralizing base in the fermentation process involving KJ122 strain of *E. coli,* succinic acid accumulates at the end of fermentation in the form of diammonium succinate. According to the present invention, one could follow several different approaches to recover succinic acid from fermentation broth containing succinic acid salt and its subsequent chemical conversion into desired chemical molecules such as BDO, GBL, and THF through appropriate chemical reactions.

The fermentation broth containing succinic acid in the form of a salt is subjected to centrifugation and appropriate filtration steps to get rid of most of the particulate material in the fermentation broth including the cell mass and proteins.

The centrifugation and filtration steps are referred as clarification step and the fermentation broth after clarification step is referred as clarified fermentation broth. In the recovery of succinic acid from fermentation broth containing a salt of succinic acid, an acidification step is followed. To convert the dilute solution of ammonium succinate to succinic acid, phosphoric acid as a proton source is provided. Generally, this can be also achieved by using an ion-exchange resin (not within the scope of the invention).

When an ion-exchange resin is used in the recovery of succinic acid from fermentation broth containing salt of succinic acid, the succinate salt is split on the surface of the resin and depending on the charge on the resin either the succinate or its counter cation is retained on the surface of the resin. For example when cation ion-exchange resin is used and the resin is charged with proton, the proton from the resin is swapped with the succinate counter cation and succinic acid comes out in the effluent.

When the clarified fermentation broth containing a salt of succinic acid is acidified with a strong acid, free succinic acid and the corresponding salt accumulate. For example, when the fermentation broth containing ammonium succinate is acidified with sulfuric acid (not within the scope of the invention), free succinic acid and ammonium succinate accumulate in the fermentation broth. Succinic acid needs to be separated from ammonium sulfate and the remaining solution, which primarily contains water and other impurities such as unconverted sugars, amino acids, and inorganic nutrients. There are several technologies that can be used to further purify the succinic acid.

When a clarified fermentation broth containing ammonium succinate is acidified with a strong acid such as sulfuric acid (not within the scope of the invention) or phosphoric acid, succinic acid and a corresponding ammonium salt such as ammonium sulfate or ammonium phosphate accumulate in the aqueous phase. The aqueous phase is concentrated preferably under vacuum to remove water and volatile carboxylic acids such as acetic acid and formic acid. The solution is concentrated to above 20 wt% of succinic acid and the crystallization of succinic acid is carried out by reducing the temperature of the mixture until its solubility limit at the corresponding temperature is lower than succinic acid concentration. While ammonium sulfate is highly soluble in aqueous solution (Figure 5), the solubility of succinic acid in water is highly dependent on temperature. Succinic acid solubility decreases with the decrease in temperature. By means of controlling the cooling rate of the aqueous solution containing succinic acid, the solubility of succinic acid can be controlled leading to controlled crystallization of succinic acid. This crystallization step resulting in the formation of primarily crystalline succinic acid from the acidified fermentation broth is referred as "first crystallization" step (Figure 2). The final slurry is filtered and the succinic acid crystals are dried to remove moisture. If necessary, the succinic acid crystals can be dissolved and recrystallized to improve the purity of the succinic acid.

There are two essential steps for particle formation by crystallization namely nucleation and crystal growth. The kinetic processes of nucleation and crystal growth require super saturation. However, excessive super saturation usually leads to large amount of fines and bad crystal morphology which negatively impact downstream processing steps such as centrifugation/filtration, drying, milling, solids-handling and means of preventing product caking. Larger surface area of fines often entraps more mother liquor with impurities. Increasing requirements for higher product purity, crystal size distribution and crystal morphology have placed new demands on crystallization process. Controlling super saturation level during crystallization is a key factor in determining the crystal morphology. Crystallization is most commonly conducted using evaporation or controlled cooling. In this invention a method is provided to utilize pH as a tool to control crystallization process.

In a normal process for recovering succinic acid from a fermentation broth comprising succinic acid salt, the acidification of the fermentation process is carried out in a single step to yield succinic acid for crystallization process. In this normal process for acidification, the target pH of 2.0 to 2.4 is achieved in a single step followed by the crystallization of resulting succinic acid. In the instant invention, a two-stage acidification of the fermentation broth comprising succinic acid is provided to control the super saturation level during crystallization process. In the first stage of acidification process according to the present invention, the pH of the fermentation broth is reduced to pH 2.5 to 3.5 followed by first crystallization step. Subsequently the pH of the fermentation broth is further reduced to pH 2.0 to 2.5 followed by second crystallization step (Figure 3). In another aspect of the present invention, it is possible to carry out the acidification in more than two stages to further improve the quality of succinic acid crystals formed. Acidification leading to pH adjustment can be done within the crystallizer or in the dissolution loops of crystal fines. If acidification is done in the fines dissolution loop, the exothermal reaction resulting from acidification process will help to dissolve crystal fines instead of using steam.

The succinic acid crystals are separated from mother liquor using centrifugation. The solid succinic acid crystal is dissolved in methanol to remove any remaining ammonium sulfate. Ammonium sulfate has a much lower solubility in methanol and can be recovered as solid precipitate from a methanolic solution. The succinic acid is fully dissolved in methanol. Succinic acid in methanolic solution is subjected to esterification reaction leading to the formation of dimethyl succinate which is further purified by fractional distillation.

The mother liquor obtained after the removal of solid crystalline succinic acid is further concentrated through evaporation at the elevated temperature under vacuum and subjected to crystallization reaction at lower temperature to precipitate ammonium sulfate. This crystallization step is referred as "second crystallization" step. Depending on the level of succinic acid remaining in the mother liquor after first crystallization step, the ammonium succinate precipitate resulting from the second crystallization step may have certain level of succinic acid crystals. Succinic acid in the ammonium sulfate precipitate can be recovered by means of dissolving precipitate from second crystallization reaction in methanol. While succinic acid is fully soluble in methanol, ammonium sulfate is relatively insoluble in methanol. Ammonium sulfate is recovered as a white precipitates while succinic acid remains fully dissolved in methanol. The ammonium sulfate obtained from methanol dissolution step can be used as a fertilizer or subjected to thermal degradation to produce ammonia and sulfuric acid which can be recycled into the process for recovering succinic acid from fermentation broth (Figure 4).

Herein, as phosphoric acid is used as an acidifying agent, ammonium phosphate is recovered as a co-product which can be used as a fertilizer or subjected to thermal degradation to yield phosphoric acid used in the process for recovering succinic acid from fermentation broth containing ammonium succinate (Figures 6A, 6B, 7 and 8).

The succinic acid recovered from fermentation broth through acidification process as described above is subjected to further downstream chemical processing as described below to obtain desired chemicals such as BDO, GBL and THF. The process begins with the esterification of succinic acid in the presence of an esterification catalyst. In one aspect of the present invention, the esterification is carried out in the absence of any exogenous esterification catalyst and such an esterification process is referred as autocatalytic esterification reaction.

Dimethyl carbonate (DMC) has become very attractive alkylation agent in the recent years as nontoxic and more environmentally benign alternative. DMC can be used for esterification of carboxylic acids instead of methanol at similar reaction performance levels. The byproduct of succinic acid esterification with DMC is $CO_2$, which can be recycled to succinic acid fermentation.

The esterification catalyst suitable for the present purpose may be a heterogeneous or homogeneous catalyst. The esterification reaction can be run using a variety of catalysts that include; zeolites (X, Y, L, beta, ZSM-5, etc.), crystalline and amorphous metal oxides (silica and alumina), alkali modified zeolites (Na, K, Li, Cs, Ru, La, etc.), anion modified metal oxides and zeolites ($SO_4$, $PO_4$, $BO_3$, etc.), cation resins (Amberlyst-15, Amberlyst-70 etc.), alkaline hydroxides (NaOH, $NH_4OH$, KOH, etc.) and alkaline alkoxides. The succinic acid ester thus produced can be purified further through fractional distillation. In one embodiment, butyl ester of succinic acid is produced first. Other types of esters such as methyl, ethyl and propyl esters of succinic acid can be obtained from butyl ester of succinic acid through transesterification process. When the esterification is done at the industrial scale, it may require the use of certain heterogeneous esterification catalysts. Under those conditions it is necessary to make sure that succinic acid feedstock to be used in the esterification reaction in the presence of such heterogeneous catalysts does not have contaminants that could interfere with the functioning of the esterification catalysts. For example, certain resins used in the industrial scale esterification of carboxylic acids are known to be sensitive to certain cation and anion contaminants of the carboxylic acid substrate and therefore it is necessary to make sure that the crystalline succinic acid used as the substrate in the esterification reaction is free of any contaminants that could interfere in the esterification reactions of the present invention.

According to the present process, succinic acid is esterified with an alcohol under super atmospheric pressure and at an elevated temperature substantially between 100°C and 300°C. When a lower monohydric alcohol is used, the pressure should be high enough to minimize its vaporization, which would otherwise occur at such temperature, so that the reactions are kept in the liquid state in the reaction zone. A number of alcohols may be used in this esterification process, especially the methyl, ethyl, isopropyl, butyl, hexyl, octyl, and similar monohydric aliphatic compounds. It is preferable to run the reaction autocatalytically or use a homogeneous catalyst in the esterification reaction. Heterogeneous acid catalysts can be subjected to fouling when in contact with succinic acid stream that has not been purified to remove cations. Sulfuric acid, phosphoric acid, hydrochloric acid, and organic acids such as alkanesulfonic acid and arylsulfonic acids are suitable homogeneous catalysts. Water formed in the esterification reaction is removed by allowing the hot reaction mixture to pass from the reaction zone at elevated pressure into a zone at atmospheric pressure, whereby water is flashed off.

In another embodiment of the present invention, a novel process is provided for the recovery of succinic acid and dimethyl succinate from the fermentation broth containing ammonium succinate. In the first step of this process, fermentation broth containing ammonium succinate is concentrated 4-5 five times using evaporation at elevated temperature and mixed with methanol or ethanol leading to the precipitation of ammonium succinate. With methanol as a solvent, ammonium succinate is precipitated as pure white crystalline solid while the impurities originally present in the fermentation broth is retained in the mother liquor (Figure 9 and 10). On the other hand, with ethanol as a solvent, ammonium succinate is precipitated out along with most of the impurities originally present in the fermentation broth. As a result, the ammonium succinate containing solid fraction resulting from ethanol treatment of fermentation broth is dark colored and the mother liquor is light-colored (Figure 11).

The white crystalline ammonium succinate fraction obtained after methanol addition to the fermentation broth can be re-suspended in methanol in the presence of sufficient amount of sulfuric acid to initiate a double displacement reaction leading to the formation of succinic acid and ammonium sulfate. Ammonium sulfate being relatively insoluble in methanol solution would precipitate out of methanol phase. Succinic acid resulting from double displacement reaction would also undergo an esterification reaction in the presence of sulfuric acid as a catalyst leading to the formation of dimethyl ester which can be further purified through fractional distillation (Figure 12).

The following examples are included to illustrate certain aspects of the present invention, and should not be viewed as exclusive embodiments.

EXPERIMENTAL SECTION

General remarks

**Strain and inoculum preparations:** KJ122 (*E. coli C, ΔldhA, ΔadhE, ΔackA, ΔfocA-pflB, ΔmgsA, ΔpoxB, ΔtdcDE, ΔcitF, ΔaspC, ΔsfcA*) was used in the present invention. KJ122 was derived from *E. coli* C (ATCC 8739) strain through genetic modifications as described by Jantama *et al* (2008a; 2008b) and in the International Patent Applications published under Patent Cooperation Treaty with International Publication Nos. WO 2008/115958 and WO 2010/115067.

*E. coli* strain KJ122 is capable of fermenting 10% glucose in AM1 mineral media to produce 88 g/L succinate, normalized for base addition, in 72 hours. AM1 medium contains 2.63 g/L $(NH_4)_2HPO_4$, 0.87 g/L $NH_4H_2PO_4$, 1.5 mM $MgSO_4$, 1.0 mM betaine, and 1.5 ml/L trace elements. The trace elements are prepared as a 1000X stock and contained the following components: 1.6 g/L $FeCl_3$, 0.2 g/L $CoCl_2 \cdot 6H_2O$, 0.1 g/L $CuCl_2$, 0.2 g/L $ZnCl_2 \cdot 4H_2O$, 0.2 g/L $NaMoO_4$, 0.05 g/L $H_3BO_3$, and 0.33 g/L $MnCl_2 \cdot 4H_2O$. The pH of the fermentation broth is maintained at pH 7.0 with: 1:4 (6 N KOH: 3 M $K_2CO_3$) (1.2 N KOH, 2.4 M $K_2CO_3$) or any other suitable base including ammonium hydroxide.

In some experiments, corn steep liquor was added. It is a byproduct from the corn wet-milling industry. When compared to the yeast extract and peptone, it is an inexpensive source of vitamins and trace elements.

**Organic acid and sugar analysis:** The concentration of various organic acids and sugars were measured by HPLC. Succinic acid, sugars, and other organic acids present in the fermentation broth were analyzed on an Agilent 1200 HPLC apparatus with a BioRad Aminex HPX-87H column. A BioRad Microguard Cation $H^+$ was used as a guard column. The standards for HPLC analysis were prepared in 0.008N sulfuric acid. The HPLC column temperature was maintained at 50°C. Sulfuric acid at 0.008N concentration was used as a mobile phase at the flow rate of 0.6 ml/min. Quantification of various components was done by measuring their absorption at 210 nm. Quantification of sugars and other components was done using a refractive index detector.

**Determination of sulfur, and phosphorous content using Inductively Coupled Plasma Optical Emission Spectrometry (ICP-OES).** Samples derived from various processes according to the present invention can be tested using Inductively Coupled Plasma Optical Emission Spectrometry. The samples are diluted to less than 5% organics using 2% trace metal grade nitric acid. The ICP-OES produces a curve that ranges from 0.05 ppm to 10ppm, thus the sample is diluted so that the target molecule concentration falls between these concentrations. If the sample contains any complex matrices or insoluble liquids, they are digested or ashed prior to being dissolved. The samples are then placed in an auto sampler connected to the ICP-OES, with a quality control in the first, middle, and last position. The ICP-OES will then analyzed the standards, generate a calibration curve, and analyze the samples. The software will then calculate the ppm for each substance detected, and from there the initial concentration of the substance can be determined based upon the dilution factor when the sample was prepared.

**Determination of chlorides, sulfates, phosphates and ammonium using Ion Chromatography (IC).** The concentration of ammonium and various anions can be quantified using Ion Chromatography. Samples were analyzed using a Donex 1100 Ion Chromatography instrument with an AS-DV auto sampler. Standards from 0.04 to 30ppm are prepared in deionized water and a calibration curve is generated prior to each run. Samples are diluted so the analyte of interest falls within the calibration curve. For ammonium analysis a Dionex CS16 column is used with a CG16 guard column and a CSRS400 suppressor. 35mM Methanesulfonic acid is used as an eluent. For chloride, sulfate and phosphate analysis a Dionex AS17-HC column is used with a AG17-HC guard column and a ASRS400 suppressor. 28mM Sodium Hydroxide is used as an eluent for cation analysis. Quantification is done by measuring the conductivity response of each component.

**Determination of amino acids.** Amino acids are measured by HPLC. Samples were analyzed on an Agilent 1100 HPLC with online derivataization and a fluorescence detector. A Phenomenex Gemini C18 column is used with a phosphate buffer and solvent gradient to elute the compounds. Samples and standards are diluted with 0.1M hydrochloric acid and derivitized with OPA reagent prior to injection. Quantification of amino acid is done by measuring the response from a fluorescence detector with excitation at 338nm and emission at 450nm.

**Example 1**

**Fermentative production of succinic acid**

*E. coli* strain KJ122 was inoculated in a minimum media consisting of NBS, 100 mM MOPS, 2% glucose, 1mM $MgSO_4$, trace element and 0.1 mM $CaCl_2$ at 37°C. Once the cell density reaches OD = 5, the inoculums were transferred to a fermenter containing initial medium consisting of 25mM KH2PO4, 3 mM $MgSO_4$, 2 mM betaine, and 8 ppm of antifoam 204. Fermentation was run in a fed-batch mode with glucose as the carbohydrate source. As succinic acid was being produced, a solution of 7M $NH_4OH$ and 3M $NH_4HCO_3$ was metered into the fermentor to maintain the pH at around 6.5-7 and to provide a source for $CO_2$. After 48 hours, the fermentation was completed. Biomass was removed by a tangential flow microfiltration unit. The filtered broth composition is shown in Table 1.

**Example 2 (Reference Example, not within the scope of the invention)**

**Acidification of fermentation broth and recovery of succinic acid crystals** - **1**

Diammonium succinate fermentation broth prepared in Example 1 was used in this experiment. 49ml of 36.25N sulfuric acid was used to acidify 1200ml of fermentation broth to pH 2.0. Then 1010g of the acidified broth was evaporated in a rotary evaporator under vacuum at 70°C to obtain 330g of concentrated broth. The condensate was analyzed and found to contain 0.8 g/L of acetic acid. The concentrated broth was cooled down by a step-wise cooling profile at the rate of -5°C every 30 minutes in an orbital shaker set at 200 rpm. The crystal was filtered under vacuum to obtain 55.75g of wet crystals. The crystal was subsequently washed with 25ml of deionized water and dried overnight at 50°C. This process corresponds to the process illustrated in the Figure 2 as "Flow sheet 1". The recovery of succinic acid as the crystal was calculated to be 53%. The composition of dry crystal was analyzed and is shown in Table 2. The crystals showed very high succinic acid purity of 95.9%. The impurities consist of 0.17wt% ammonium ion and 0.22 wt% sulfur.

**Example 3 (Reference Example, not within the scope of the invention)**

**Acidification of fermentation broth and recovery of succinic acid crystals** - **2**

Diammonium succinate fermentation broth prepared in Example 1 was used in this experiment. 1038g of the broth was concentrated in a rotary evaporator to 416g under vacuum. Some brown solids precipitated out of the solution. The condensate was analyzed and was found to contain 2.8 g/L of ammonia. The concentrated broth was divided into three portions. Portion 1 (99.92g) was filtered to remove solids and then acidified with 8ml of 36.25N $H_2SO_4$ to pH 2.0. Portion 2 (84.66g) was acidified with 8ml of 36.25N $H_2SO_4$ to pH 2.0. Portion 3 was saved as a reference. During the acidification step, white precipitate started to form at pH 5.2 and more came out of solution as the pH dropped further. After letting the solution cool to ambient room temperature, the precipitates from portions 1 and 2 were filtered and each was washed with 10ml of deionized water. The solids were dried overnight at 50°C. Compositional analysis of these solids is shown in Table 2. Compared with Example 2, this process configuration uses less sulfuric acid to bring the solution pH to 2.0. This process corresponds to the process illustrated in the Figure 2 as "Flow sheet 2a". It is believed that since ammonia was removed as a condensate, less acid is required to reduce the solution pH, which is very desirable from an economic standpoint. However, the purity of the crystal was lowered. Crystal Portion 1, which had been filtered after evaporation showed 90 wt% succinic acid purity, while crystal Portion 2 showed only 82wt% purity. The filtration step after evaporation clearly helped purify the crystals substantially; the amount of ammonium ion, sulfur, phosphorus, potassium ion, and amino acid are substantially less.

**Example 4 (Reference Example, not within the scope of the invention)**

**Acidification of fermentation broth and recovery of succinic acid crystals - 3**

Diammonium succinate fermentation broth prepared in Example 1 was used in this experiment. 1010g of the broth was concentrated under reduced pressure at 70°C in a rotary evaporator to 264g. The condensate was analyzed and found to contain 2.1 g/L of ammonia. Some brown solids precipitated out of solution. However, the broth was not filtered. The solution was acidified with 34.85ml of 36.25N $H_2SO_4$ to the pH of 2.07. Then, a controlled crystallization was performed in an orbital shaker by cooling at the rate of 5°C every 30 minutes to room temperature. The crystal was filtered and washed with 40ml of deionized water and then dried at 50°C overnight. The crystal composition is shown in Table 2. By doing a proper controlled cooling crystallization step, the succinic acid crystal had a surprisingly very high purity of 99 wt% succinic acid even without a filtration step after evaporation. The amount of reduction in ammonium ion, sulfur, phosphorus, and amino acid is greater than 10 fold. This process corresponds to the process illustrated in the Figure 2 as "Flow sheet 2b".

The sulfuric acid usages and the crystal qualities from three flow sheets are compared in Table 2. Results showed that Flow sheet 2a and 2b used less sulfuric acid than that of Flow sheet 1 to adjust the pH to 2. In terms of the crystal purity, Flow sheet 2a had the highest amount of impurity incorporated into the final product. This is likely due to the fact that succinic acid precipitated out in an uncontrolled manner and the mother liquor carrying impurities was trapped inside the precipitate. With a controlled recrystallization step in Flow sheet 2b, the crystal quality was much improved. The results suggest that it is desirable to concentrate the sample first, followed by acidification and crystallization.

**Example 5**

**Effect of temperature on succinamic acid formation**

A 10 wt% aqueous solution of synthetic diammonium succinate was prepared by dissolving reagent grade succinic acid in water and then aqueous ammonia was added to the solution. 45ml of the solution was charged under atmospheric pressure into a 75ml vessel of Multi-Parr reactor model 5000. The solution was heated up to various temperatures and held for a period of time. After that the solution was immediately cooled down by running cooling water through a cold finger. Then the reactor content was emptied and the composition of the product was analyzed. The results are shown in Table 3. These results showed that increasing temperature and the exposure duration can increase the formation of amide by-products. The process according to this invention recommends the evaporation process to be conducted under reduced pressure, which should be advantageous in reducing the yield loss of succinic acid to amides.

**Example 6**

**Crude crystallization of succinic acid in large volume fermentation broth**

Liquid samples 1 and 2 were obtained from a large volume fermentation (85,000 liters) run using KJ122 strain as described in the Example 1. Liquid sample 1 is diammonium succinate fermentation broth after centrifugation to separate cell mass. Liquid sample 2 is diammonium succinate fermentation broth after centrifugation and an acidification step with sulfuric acid to pH 4.5. Approximately 45kg of each liquid sample was used to generate crude succinic crystal using the following procedures: (1) approximately 45kg of each sample was concentrated ~3x via evaporation at reduced pressure. (2) Concentrated ammonium succinate was filtered by a filter press using 5-micron and 0.5-micron membranes. (3) The filtered material was then partitioned into 4kg batches for acidification to pH≈2 with 96% $H_2SO_4$; this was conducted in 5L Pyrex bottles using a stir bar for agitation. (4) Heating and recrystallization was executed in an Innova-43 incubator shaker at 150 rpm and an initial temperature of 70°C. The incubator shaker was programmed to drop the temperature by 10°C per hour in order to carry out slow cooling. (5) Precipitated crystals and mother liquor from each acidification/crystallization batch were pooled together in a 20L Jacketed Filter Reactor to accomplish filtration. Vacuum was used to pull mother liquor through a Teflon filter into a container. Crystals were washed with deionized water and then left to dry for several hours under vacuum. (6) Afterwards, the crystals were harvested from the reactor. Mass balance is summarized in Table 4. The compositions of the crude crystals are shown in Tables 5 and 6.

**Example 7**

**Acidification of fermentation broth and recovery of succinic acid crystals**

Diammonium succinate fermentation broth prepared in Example 1 was used in this experiment. 36.25N sulfuric acid was used to adjust the broth down to pH 2.4. The pH adjusted broth was then passed through a 0.5 micron filter. Then 1600mL of the acidified broth was evaporated in a rotary evaporator under vacuum at 70°C to obtain 440mL of concentrated broth. The condensate was analyzed and found to contain 8 g/L of acetic acid. The concentrated broth was cooled down by a step-wise cooling profile at the rate of -5°C every 30 minutes in an orbital shaker set at 200 rpm. The crystal was filtered under vacuum to obtain 74.2g of wet crystals. The crystal was subsequently washed with 25ml of deionized water and dried overnight under vacuum. The recovery of succinic acid as the crystal was calculated to be 73%. The composition of dry crystal was analyzed and is shown in Tables 7. The crystals showed very high succinic acid purity of 99%.

**Example 8**

**Recovery of ammonium sulfate from mother liquor using methanol**

Diammonium succinate fermentation broth prepared in Example 1 was used in this experiment. Succinic acid crystals were recovered following the procedure described in Example 7. The filtrate after removing the succinic acid crystals contained 423g of mother liquor. This mother liquor was evaporated in a rotary evaporator under vacuum at 70°C to obtain 215g of concentrated slurry. The slurry was filtered under vacuum to obtain 83g of wet crystals. The wet crystals had a composition of 15 wt% succinic acid and 72wt% ammonium sulfate. The wet crystals were mixed with 200mL methanol and filtered under vacuum to obtain 54g of crystals. The composition of crystals and methanol filtrate were analyzed and the results are shown in Table 7. The final AMS crystals showed a high ammonium sulfate concentration with <1% impurities, 0.2% succinic acid.

## Example 9

### Formation of methyl succinate esters form methanol filtrate

The methanol filtrate from Example 8 was combined with 20 g of Amberlyst 36 and heated for 6hrs at 60C under atmospheric pressure. After heating, the final liquid was analyzed and the composition was 71g/L dimethyl succinate, 14g/L monomethyl succinate and 0.5 g/L succinic acid.

## Example 10

### Acidification of Ammonium succinate by H3PO4 and recovery of succinic acid crystals

Diammonium succinate solution prepared by dissolving 258 g/L ammonium succinate (equivalent to 200 g/L SAC) at 60°C was titrated with concentrated phosphoric acid to pH2.0, 2.5, and 3.0 and a controlled crystallization was performed in an orbital shaker by cooling at the rate of -5°C every hour to room temperature. The resulting succinic acid crystal was filtered and analyzed. The composition of succinic acid crystal resulting from acidification with phosphoric acid is shown in Table 8. As a result of following a controlled cooling crystallization procedure, the resulting succinic acid crystal had a very little ammonium diphosphate (ADP) even without a water rinsing step. Since ADP has high solubility in water ($\sim$ 55% at 25 C), mother liquor can retain approximately half its weight of ADP. Water rinsing of succinic acid crystals should be applied as it will improve the quality of succinic acid crystals.

Since phosphoric acid has three pKa values, i.e. $pKa_1 = 2.1$, $pKa_2 = 7.2$, $pKa_3 = 12.3$, Succinic acid has two $pKa_1 = 4.2$, $pKa_2 = 5.6$, theoretically only 1st proton of H3P04 can be used to protonate succinate. Titration curve (Figure 7) clearly shows that double amount of $H_3PO_4$ to succinic acid are needed to bring diammonium succinate from neutral pH to pH 2.5. Analytical results confirmed phosphate generated in solution is ammonium dihydrogen phosphate (ADP).

Table 8 shows results of recovery of succinic acid crystals from phosphate solution at pH 2, pH2.5 and pH3. Free succinic acid has much lower solubility in water than its diammonium salt. When pH is 1 unit below the 1st pKa of an organic acid, about 10% of the organic acid is in its salt form. If pH is 2 units below the 1st pKa of an organic acid, only about 1 % of the organic acid is in its salt form. Since the 1st pKa value of succinic acid is 4.2, if solution is acidified to 2.2, 99% of succinic acid will be in free acid form, which will be much easier to crystallize. Table 8 show that when crystallization is performed at pH 3.0, 10.18 g of succinate still remained in solution and only 42 % crystallization yield was achieved. However when pH values were adjusted to 2 and 2.5, the SAC crystal yield were 69% and 58 % respectively. For these reasons, it is suggested that it is better to maintain industrial scale crystallization of succinic acid preferentially around the pH range of pH 2.0 to pH2.5.

## Example 11

### Salting out and solid formation

2L fermentation broth was concentrated in a rotary evaporator, under vacuum at. The chemical composition of feed introduced into the evaporator, concentrate recovered from evaporator and the condensate from evaporator is shown in Table 9. The condensate stream showed 2.96 g/l of NH4+ ion and <10 ppm of other ions. The loss of NH4+ ion to the condensate stream was observed to be approximately 9% by wt. The concentrate stream showed small formation of Succinimide by-product.

50 ml sample from the concentrate recovered from evaporator was used for the salting out process in the next step. This 50 ml sample from concentrate recovered from evaporator had 32.19 g of diammonium succinate. Methanol was used for precipitation of solids out of the concentrate recovered from evaporator. Various weight ratios of methanol to diammonium succinate theoretically present in the concentrate recovered from evaporator were tested to understand the effectiveness of precipitation under different solvent loadings. This step is referred to as solvent precipitation or salting out insolubles using a solvent. This method is aimed at exploiting the low solubility of ammonium salts in alcohol.

The methanol:diammonium succinate broth weight ratios 2x, 3x and 4x were tested in three different glass beakers. 3 glass beakers were used with different amount of methanol concentrations 64.38g, 96.57g & 128.77g representing 2x, 3x & 4x ratios respectively was added to 50 ml of concentrate recovered from evaporator and the solutions were stirred on a hot plate at room temperature for 2 hours. This time was sufficient to precipitate out solids from the diammonium succinate solution resulting in slurry that was separated into solid and liquid stream using 0.22 micron Nalgene® vacuum filters. Solids and liquids were weighed and analyzed. Masses of solids collected for samples with 2x, 3x, & 4x ratio were 26.64g, 32.08g & 22.18g respectively whereas the masses of filtrate collected for samples with 2x, 3x & 4x ratios were 93.76g, 120.17g & 160.14g respectively. This filtrate can be sent to a distillation step for the recovery of methanol. Water can also be recovered or recycled back to dilute diammonium succinate stream before concentration step.

From compositional analysis it was determined that a single-pass recovery of diammonium succinate broth in the precipitation step was 64%, 62% & 60% for methanol: diammonium succinate weight ratios 2x, 3x and 4x respectively (Table 10 and 11). These recovery numbers have been observed for a single-pass precipitation without recycling the filtrate, they should improve as the recycle ratios are increased.

Solids collected in the previous precipitation step from methanol: diammonium succinate weight ratios 2x, 3x and 4x experiments were combined together to make up the feed for the next step known as the salt splitting reaction step. In this step, diammonium succinate solid is re-suspended in methanol, the reaction is catalyzed by using a mineral acid to split the diammonium succinate salt into an succinic acid and an inorganic salt. In our case we suspended diammonium succinate salt in methanol in presence of 98% sulfuric acid which splits the ammonium succinate salt to form succinic acid and ammonium sulfate (AMS). AMS is an inorganic salt that has low solubility in methanol and therefore it precipitates out of the solution. Succinic acid released during the salt splitting step undergoes esterification in the presence of sulfuric acid as a catalyst leading to the formation of dimethyl succinate.

## Example 12

### Succinic acid crystallization by two-stage acidification process

Fermentation broth comprising ammonium succinate was subjected to centrifugation and evaporation followed by an ultrafiltration step. The permeate from ultrafiltration step was concentrated to 250.4 g/L succinic acid equivalent and subjected to either one-stage or two-stage acidification process. Under one-stage acidification process, the pH of the fermentation broth was brought down to pH 2. 2 to 2.4 in a single step and the resulting precipitate was redissolved by elevating the temperature to 80°C. Subsequently the temperature of the acidified fermentation broth was gradually lowered to 30°C at the rate of 5°C/hour to initiate the crystallization process. Under two-stage acidification process, the same protocol for crystallization was followed except the acidification was carried out in two stages. In the first stage of two-stage acidification process, the pH of the fermentation broth was lowered to pH 2.8 - 3.0 followed by a crystallization step. In the second stage of two-stage acidification process, the pH of the fermentation broth was further lowered to pH 2.2 - 2.4 followed by a crystallization step. After crystallization completed, slurries from one-stage acidification process and two-stage acidification process were filtered at using the same type Nalgene filters (Disposable 2.0 $\mu$m vacuum filters) under vacuum. Crude succinic acid crystals from one-stage acidification process and two-stage acidification process were submitted for analysis without any washing. Both experiments were done in triplicates. The results from these experiments are provided in Table 12. Figure 13A provides a comparison of yield and purity of the succinic acid crystals from one-stage acidification process and two-stage acidification process. Figure 13B provides the comparison of ammonium and sulfur impurities in the succinic acid crystals obtained through one-stage acidification process and two-stage acidification process. Figure 13C provides the comparison of level of aspartic acid, glutamic acid and alanine in the succinic acid crystals resulting from one-stage acidification process and two-stage acidification process. In the Figures 13A, 13B and 13C, the one-stage acidification process is referred as "Normal Crystallization" process

## Example 13

### Succinic acid crystallization by two-stage acidification process -Effect of salt concentration

To further understand the effect of initial concentration of ammonium succinate on the crystallization process under one-stage and two-stage acidification process, fermentation broth comprising ammonium succinate was subjected to centrifugation and evaporation followed by an ultrafiltration step. The permeate from ultrafiltration step contained 66 grams of succinic acid salt per liter. This permeate from ultrafiltration step was concentrated up to six different concentrations (184, 210, 255, 285, 316, and 360 [g/L]) using Rotovap operated around 80-90 RPM and at 70-80°C. All these six samples were subjected to one-stage or two-stage acidification process followed by a crystallization process as described in the Example 12 above and each sample was tested under two acidification procedures: 1 pH-stage (normal) and 2 pH-stage (new) crystallization. The testing was done in triplicates.

After crystallization process is complete, the resulting slurry was filtered using a vacuum Nalgene filters (2.0 $\mu$m) at room temperature. About 1.2g samples of the wet crude crystals were washed using a 1:1 (wt/wt) ratio of room temperature deionized water using Millipore's low-binding Durapore PVDF membrane (5.0 $\mu$m) centrifuge tube in the tabletop Beckman Accuspin centrifuge operated at ~1000 g. Each sample was washed with half of the DI water for two minutes followed by a second wash with the other half of DI water for another two minutes. The two minutes duration included start-up spin time but excluded time taken to reach zero RPM. As the results shown in Figure 14, with the increase in the concentration of succinic acid salt in the starting material, the two-stage acidification process showed higher yield for succinic acid crystals. In the Figure 14, the one-stage acidification process is referred as "Normal Crystallization".

**Example 14**

**Crystal particle size comparison**

Based on the results from the experiments in Example 13 above that fermentation broth having higher succinic acid salt concentration would give higher yield for succinic acid crystals, a fermentation broth having 300 g of succinic acid salt/L was chosen to produce roughly 0.7kg of succinic acid crystal either using one-stage acidification process or two-stage acidification process. The succinic acid crystal obtained from both one-stage acidification process (1S) and two-stage acidification process (2S) were subjected to compositional and particle size distributional analysis. Figure 15A provides the level of ammonium and sulfur impurities in the succinic acid crystals obtained from one-stage acidification process (1 stage) and two-stage acidification process (2pH stages). Figure 15B provides the level of various amino acid impurities in the succinic acid crystals obtained from one-stage acidification process (1 stage) and two-stage acidification process (2pH stages). Figure 15C provides the particle size distribution of succinic acid crystals obtained from one-stage acidification process (1S) and two-stage acidification process (2S). The SAC crystals obtained using two-stage acidification process had less fines with the particle size of 125 microns or lesser. In general, the two-stage scidificaiton process yielded crystals distributed around 300 to 500 micron range. In addition, the bell-shaped curve for crystal distribution under two-stage acidification process was found narrower suggesting the crystal size distribution under two-stage acidification process was more uniform. As a result there were lesser impurities as they are washed away earlier in the process.

**Example 15**

**Ammonium sulfate production using controlled evaporation and crystallization**

Fermentation broth containing ammonium succinate was produced using the fermentation process described in Example 1. This broth was passed through an ultra filtration unit and used to produce succinic acid crystals using two-stage pH controlled crystallization as described in Example 12. The mother liquor from the succinic acid crystallization had a composition of 54 g/L succinic acid, 47 g/L acetic acid, 270 g/L sulfate, 123 g/L ammonium and 13 g/L phosphate. This material then had 20% of the volume evaporated in a rotary evaporator. The remaining slurry was heated to 80°C and slowly cooled at 5 degrees Celsius per hour to 30°C. The crystals were then filtered and dried under vacuum at room temperature. The composition of the crystals was >98 wt% ammonium sulfate with approximately 1% succinic acid.
The ammonium sulfate crystals produced following this procedure do not need to undergo a further washing step. The filtrate can then be recycled to either the first or second evaporation step in the process.

| Table 1. Composition of Fermentation Broth | |
|---|---|
| Succinic Acid (g/L) | 78.2 |
| Acetic Acid (g/L) | 0.6 |
| Lactic Acid (g/L) | - |
| Pyruvic Acid (g/L) | 0.04 |
| Malic Acid (g/L) | - |
| Fumaric Acid (g/L) | 0.06 |
| Formic Acid (g/L) | - |
| Ammonium ion (g/L) | 23.0 |
| Chloride ion (ppm) | 2.5 |
| Total sulfur (ppm) | 110 |
| Total phosphorus (ppm) | 318 |

(continued)

| Table 1. Composition of Fermentation Broth | |
|---|---|
| Amino acids (g/L) | 22.6 |

| Table 2. Product quality of succinic acid crystals | | | | |
|---|---|---|---|---|
| | Flow sheet 1 | Flow sheet 2a | | Flow sheet 2b |
| Process Configuration | Acidify → Evaporate → Crystallize | Evaporate → Filter → Acidify & Precipitate | Evaporate → Acidify & Precipitate | Evaporate → Acidify & Precipitate → Recrystallize |
| $H_2SO_4$ usage g/g broth | 0.0746 | 0.0639 | | 0.0635 |
| - Moisture (wt%) | 0.24% | 0.21% | 0.22% | 0.42% |
| - Succinic acid (wt%) | 95.9% | 90.0% | 82.3% | 99.2% |
| - Fumaric acid (wt%) | 0.07% | 0.06% | 0.05% | 0.11% |
| - Succinamic acid (wt%) | 0.18% | 0.67% | 0.73% | 0.42% |
| - Succinimide (wt%) | not detected | not detected | not detected | not detected |
| - Succindiamide (wt%) | not detected | not detected | not detected | not detected |
| - Amino acids (wt%) | 0.39% | 3.86% | 4.51% | 0.14% |
| - Ammonium (ppmw) | 1769.8 | 11411 | 17751 | 130.6 |
| - Sulfur (ppmw) | 2248.8 | 19308 | 23631 | 1082.8 |
| - phosphorus (ppmw) | 76.0 | 825.9 | 915.3 | not detected |
| - Chloride (ppmw) | not detected | 8.45 | 7.45 | not detected |
| - Calcium (ppmw) | 22.6 | 3.6 | 4.1 | not detected |
| - Potassium (ppmw) | 612.0 | 272.3 | 432.7 | 29.94 |
| - Magnesium (ppmw) | 12.5 | 4.5 | 7.0 | not detected |
| - Sodium (ppmw) | 7.8 | 22.9 | 33.6 | not detected |

| Table 3. Effect of temperature on succinamic acid formation | | | | | |
|---|---|---|---|---|---|
| Run Number | MA13 | MA15 | MA16 | MA17 | MA18 |
| Temperature (°C) | 150 | 120 | 120 | 90 | 90 |
| Duration (hrs) | 4 | 4 | 8 | 4 | 8 |

(continued)

| Yield | | | | | |
|---|---|---|---|---|---|
| - Succinimide | 0.13% | 0.04% | 0.13% | 0.00% | 0.00% |
| - Succindiamide | 0.08% | 0.00% | 0.00% | 0.00% | 0.00% |
| - Succinamic acid | 10.7% | 0.7% | 1.6% | 0.00% | 0.07% |

| Table 4. Mass balance for succinic acid samples 1 and 2 obtained from crude crystallization of large volume fermentation broth | | | | | |
|---|---|---|---|---|---|
| | Liquid feed (kg) | SAC g/L in feed | Total SAC in feed (kg) | Total Crude crystal (kg) | SAC in crude crystal (kg) | % SAC recovered |
| Sample 1 | 42.5 | 68.38 | 2.84 | 2.32 | 2.18 | 76.8% |
| Sample 2 | 45.2 | 68.09 | 3.00 | 2.72 | 2.59 | 86.3% |

| Table 5. Composition of crude succinic crystals | | | |
|---|---|---|---|
| | | Crude 1 | Crude 2 |
| Color (YI) | | 19.51 | 15.83 |
| Color (WI) | | -7.39 | 1.46 |
| Moisture | wt % | 6.47% | 0.71% |
| **Organics** | | | |
| Succinic acid | wt % | 95.91% | 96.98% |
| Acetic Acid | wt % | 0.092% | 0.075% |
| Pyruvic Acid | wt % | 0.006% | 0.028% |
| Malic Acid | wt % | not detected | not detected |
| Fumaric Acid | wt % | 0.004% | 0.009% |
| Lactic Acid | wt % | not detected | not detected |
| Glucose | wt % | 0.016% | 0.019% |
| Maltose | wt % | 0.050% | 0.030% |
| Glycerol | wt % | not detected | not detected |
| Formic Acid | wt % | not detected | not detected |
| Succinamic Acid | wt % | 0.03% | 0.05% |
| Succinimide | wt % | not detected | not detected |
| Succindiamide | wt % | not detected | 0.02% |

| Table 6. Composition of crude succinic crystals (Anions and Cations) | | | |
|---|---|---|---|
| | | Crude 1 | Crude 2 |
| **Anions** | | | |
| Sulphate | ppm | 11267.51 | 9053.58 |
| Phosphate | ppm | not detected | not detected |

(continued)

| Table 6. Composition of crude succinic crystals (Anions and Cations) | | | Crude 1 | Crude 2 |
|---|---|---|---|---|
| **Anions** | | | | |
| Chlorides | ppm | | 3.39 | 0.51 |
| **Cations** | | | | |
| Ammonium | ppm | | 5747.57 | 4650.70 |
| As | ppm | | not detected | not detected |
| Ca | ppm | | 1.73 | 0.91 |
| Cu | ppm | | 0.52 | not detected |
| Fe | ppm | | not detected | 1.12 |
| K | ppm | | 143.30 | 100.75 |
| Mg | ppm | | 9.18 | 6.22 |
| Mn | ppm | | not detected | not detected |
| Ni | ppm | | not detected | not detected |
| Pb | ppm | | not detected | not detected |
| Zn | ppm | | 0.39 | not detected |
| Na | ppm | | 2.13 | 3.68 |
| Cr | ppm | | not detected | not detected |
| S | ppm | | 4264.90 | 3471.00 |
| P | ppm | | 102.72 | 74.14 |
| Total Amino Acid | ppm | | 253.1 | 181.18 |

| Table 7 Com position analysis of various fractions from the process flow diagram shown in Figure 1 | | | | | | | |
|---|---|---|---|---|---|---|---|
| | Feed | Crude Succinic Crystals | ML#1 | Mixed Solids | ML #2 | MeOH Solution | AMS |
| Appearance | | Light Brown | | Yellow | | | White |
| Succinic g/L or wt% | 70.10 | 99.30% | 56.18 | 15.58% | 50.21 | 61.86 | 0.22% |
| Maltose g/L or wt% | 1.69 | not detected | 6.35 | 0.32% | 14.25 | 1.09 | not detected |
| Glucose g/L or wt% | 0.23 | 0.09% | 0.99 | 0.06% | 2.93 | 0.25 | not detected |
| Acetic g/L or wt% | 10.90 | 0.15% | 15.20 | 0.22% | 11.38 | 0.71 | not detected |
| Lactic g/L or wt% | 0.64 | not detected | 2.00 | 0.10% | 5.56 | 0.38 | not detected |
| Pyruvic g/L or wt% | not detected | not detected | 0.25 | not detected | 0.70 | not detected | not detected |
| Malic g/L or wt% | not detected | not detected | not detected | not detected | not detected | not detected | not detected |

(continued)

| Table 7 Com position analysis of various fractions from the process flow diagram shown in Figure 1 | | | | | | | |
|---|---|---|---|---|---|---|---|
| | Feed | Crude Succinic Crystals | ML#1 | Mixed Solids | ML #2 | MeOH Solution | AMS |
| Fumaric g/L or wt% | 0.01 | 0.02% | 0.01 | 0.00% | 0.01 | 0.01 | not detected |
| Formic g/L or wt% | not detected | not detected | not detected | not detected | not detected | not detected | not detected |
| Glycerol g/L or wt% | not detected | not detected | not detected | not detected | not detected | not detected | not detected |
| Chloride ppm | 14.5 | 21 | 115 | 518 | 4 | 0.4 | 1536 |
| Sulfate ppm | 70601 | 4775 | 249134 | 509639 | 259688 | 1325.9 | 655889 |
| Phosphate ppm | 1820 | 1747 | 7668 | 2202 | 14442 | 680.0 | not detected |
| Ammonium ppm | 29011 | 1622 | 92449 | 211396 | 100014 | 332.4 | 284484 |
| Total Amino Acids ppm | 7129.5 | 581.5 | 18346.0 | 6809.6 | 83710.7 | 2049.7 | 249.0 |

| Table 8. Recovery of Succinic Acid Crystals from H3PO4 Acidified Solution | | | |
|---|---|---|---|
| Total Solution Weight (g) | 115 | 115 | 115 |
| Total SAC in Solution (g) | 15.5 | 18.4 | 17.7 |
| Total ADP in Solution (g) | 29.9 | 43.8 | 35.1 |
| | | | |
| **pH values at Crystllization** | **pH2** | **pH2.5** | **pH3** |
| | | | |
| Wet Crystal | | | |
| Wet Crystal Collected (g) | 15.8 | 15.2 | 13.5 |
| SAC in Wet Crystal (g) | 10.7 | 10.7 | 7.5 |
| Mother Liquor Retained (g) | 2.0 | 3.0 | 4.0 |
| ADP Retained (g) | 0.9 | 1.1 | 1.56 |
| Accountable mass | 0.9 | 1.0 | 1.0 |
| Mother Liquor | | | |
| SAC in mother Liquor (g) | 4.8 | 7.7 | 10.2 |
| ADP in mother liquor (g) | 29.1 | 42.7 | 33.6 |
| | | | |
| **SAC Crystal Yield** | **69%** | **58%** | **42%** |

| Table 9. Composition of fermentation broth before and after evaporation and the condensate from the evaporator | | | |
|---|---|---|---|
| Component analyzed | Feed to the evaporator | Concentrate from the evaporator | Condensate from the evaporator |
| Ammonium ion | 27.83 (g/L) | 145.41 (g/L) | 2959 (ppm) |
| Succinic acid | 74.19 (g/L) | 498.42 (g/L) | not tested |
| Acetic acid | 8.02 (g/L) | 53.50 (g/L) | not tested |
| Lactic acid | not detected | not detected | not tested |
| Fumaric acid | not detected | 0.03 (g/L) | not tested |
| Formic acid | not detected | 0.27 (g/L) | not tested |
| Pyruvic acid | 0.07 (g/L) | not detected | not tested |
| Malic acid | 0.2 (g/L) | not detected | not tested |
| Phosphate ion | 4.02 (g/L) | 34.00 (g/L) | not tested |
| Sulfate ion | 0.19 (g/L) | 2.27 (g/L) | 5.04 (ppm) |
| Chloride ion | 0.40 (g/L) | 2.17 (g/L) | 1.04 (ppm) |
| Maltose | 4.76 (g/L) | 7.30 (g/L) | not tested |
| Glucose | 0.88 (g/L) | | not tested |
| Glycerol | 0.72 (g/L) | 6.89 (g/L) | not tested |
| Amino acids | not tested | 8.66 (g/L) | not tested |
| Succinamide | not detected | not detected | not tested |
| Succinimide | not tested | 3.20 (g/L) | not tested |

| Table 10. Composition of solid fraction after salting out process | | | |
|---|---|---|---|
| Methanol: Concnetrated Broth ratio | 2X | 3X | 4X |
| Mass of sample (g) | 0.34 | 0.36 | 0.38 |
| Succinic acid (wt %) | 56.4 | 44.78 | 62.76 |
| Acetic acid (wt %) | 0.89 | 1.11 | 0.53 |
| Ammonium ion (wt %) | 21 | 17 | 24 |
| Other organic acids (wt %) | 0.01 | not detected | 0.01 |
| Total sugars (wt %) | not detected | not detected | not detected |
| Methanol (wt %) | 9 | 19 | 3 |
| Glycerol (wt %) | not detected | not detected | not detected |

| Table 11. Composition of liquid fraction after salting out process | | | |
|---|---|---|---|
| Methanol: Concentrated Broth ratio | 2X | 3X | 4x |
| Succinic acid (g/L) | 91.3 | 64.6 | 54 |
| Acetic acid (g/L) | 21.1 | 15.6 | 12.1 |
| Ammonium ion (ppm) | 23395 | 17246 | 12569 |
| Other organic acids (ppm) | 0.49 | 0.19 | 0.28 |
| Total sugars (g/L) | 2.08 | 1.59 | 1.51 |

(continued)

| Table 11. Composition of liquid fraction after salting out process | | | |
|---|---|---|---|
| Methanol (g/L) | 527.3 | 588.6 | 642.8 |
| Glycerol (g/L) | 3.1 | 1.9 | 1.7 |

(continued)

| Table 12 Comparison of one-stage and two-stage acidification processes | | | | | | | | | | |
|---|---|---|---|---|---|---|---|---|---|---|
| | Normal 1pH Stage Crystallization | | | | | 2 pH Stage Crystallization | | | | |
| Flaks | A | B | C | Average | Std Dev | A | B | C | Average | Std Dev |
| Filter (g) | 35.71 | 35.50 | 35.28 | | | 35.35 | 35.61 | 35.41 | | |
| Bottle (g) | 50.57 | 50.78 | 50.64 | | | 50.79 | 50.89 | 50.58 | | |
| Filter +Wet Crystal (g) | 63.84 | 62.17 | 61.98 | | | 59.91 | 61.46 | 58.77 | | |
| Bottle +ML (g) | 155.23 | 156.71 | 156.16 | | | 160.04 | 157.59 | 158.88 | | |
| Mother Liquor (g) | 104.66 | 105.93 | 105.52 | 105.37 | 0.65 | 109.25 | 106.70 | 108.30 | 108.08 | 1.29 |
| Wet Crystal (g) | 28.13 | 26.67 | 26.70 | 27.17 | 0.83 | 24.56 | 25.85 | 23.36 | 24.59 | 1.25 |
| Moisture | 15.00% | 14.46% | 17.40% | 0.16 | 0.02 | 12.44% | 17.25% | 14.66% | 0.15 | 0.02 |
| SAC Content | 69.3% | 69.8% | 64.1% | 67/7% | 0.03 | 76.1% | 78.2% | 82.9% | 79.1% | 0.03 |
| SAC Yield | 77.8% | 74.3% | 68.4% | 73.5% | 0.05 | 74.6% | 80.8% | 77.3% | 77.6% | 0.03 |
| Dried Basis Purity | 81.5% | 81.6% | 77.6% | 80.2% | 0.02 | 86.9% | 94.6% | 97.1% | 92.8% | 0.05 |

All references are listed for the convenience of the reader.

U.S. Patent No. 2,565,487
U.S. Patent No. 2,722,541
U.S. Patent No. 2,766,273
U.S. Patent No. 4,100,189
U.S. Patent No.4,405,717
U.S. Patent No. 4,444,881
U.S. Patent No. 4,645,855
U.S. Patent No. 4,885,247
U.S. Patent No. 5,034,105
U.S. Patent No. 5,068,418
U.S. Patent No. 5,132,456
U.S. Patent No. 5,143,834
U.S. Patent No. 5,168,055
U.S. Patent No. 5,210,296
U.S. Patent No. 5,323,639
U.S. Patent No. 5,352,825
U.S. Patent No. 5,405,992
U.S. Patent No. 5,449,824
U.S. Patent No. 5,453,365
U.S. Patent No. 5,502,240
U.S. Patent No. 5,510,526
U.S. Patent No. 5,641,406
U.S. Patent No. 5,766,439
U.S. Patent No. 5,773,653
U.S. Patent No. 5,780,276
U.S. Patent No. 5,780,678
U.S. Patent No. 5,786,185
U.S. Patent No. 5,892,109
U.S. Patent No. 5,958,744
U.S. Patent No. 5,986,133
U. S. Patent No. 6,043,392
U.S. Patent No. 6,087,532
U.S. Patent No. 6,160,173
U.S. Patent No. 6,187,951
U.S. Patent No. 6,229,046
U.S. Patent No. 6,265,190
U.S. Patent No. 6,280,985
U.S. Patent No. 6,291,708
U.S. Patent No. 6,319,382
U.S. Patent No. 6,320,077
U.S. Patent No.6,342,626
U.S. Patent No. 6,384,276
U.S. Patent No. 6,403,825
U.S. Patent No. 6,452,0151
U.S. Patent No. 6,472,559
U.S. Patent No.6,476,239
U.S. Patent No. 6,478,965
U.S. Patent No. 6,509,179
U.S. Patent No. 6,518,454
U.S. Patent No. 6,534,079
U.S. Patent No. 6,583,310
U.S. Patent No. 6,660,505
U.S. Patent No. 6,641,734
U.S. Patent No. 6,664,413
U.S. Patent No.6,670,505
U.S. Patent No. 6,772,213

U.S. Patent No. 6,803,217
U.S. Patent No. 6,872,314
U.S. Patent No. 6,926,810
U.S. Patent No. 6,942,803
U.S. Patent No. 7,019,170
U.S. Patent No. 7,026,145
U.S. Patent No. 7,144,977
U.S. Patent No. 7,186,856
U.S. Patent No. 7,238,837
U.S. Patent No. 7,297,809
U.S. Patent No. 7,439,392
U.S. Patent No. 7,498,450
U.S. Patent No. 7,528,277
U.S. Patent No. 7.601,865
U.S. Patent No. 7,632,479
U.S. Patent No. 7,652,167
U.S. Patent No. 7,696,375
U.S. Patent No. 7,709,689
U.S. Patent No. 7,754,916
U.S. Patent No. 7,820,859
U.S. Patent No. 7,915,447
U.S. Patent No. 8,084,626
U.S. Patent No. 8.193,375
U.S. Patent No. 8,203,021
U.S. Patent No. 8,241,482
U.S. Patent No. 8,293,935
U.S. Patent No. 8,338,145
U.S. Patent No. 8,410,306
U.S. Patent Application Publication No. 2004/0210087
U.S. Patent Application Publication No. 2006/0252956
U.S. Patent Application Publication No. 2006/0276674
U.S. Patent Application Publication No. 2006/0041165
U.S. Patent Application Publication No. 2007/0014895
U.S. Patent Application Publication No. 2007/0096481
U.S. Patent Application Publication No. 2007/0161816
U.S. Patent Application Publication No. 2007/0167649
U.S. Patent Application Publication No. 2008/0287714
U.S. Patent Application Publication No. 2008/042958
U.S. Patent Application Publication No. 2009/0076297
U.S. Patent Application Publication No. 2009/0104322
U.S. Patent Application Publication No. 2009/0234160
U.S. Patent Application Publication No. 2010/0297715
U.S. Patent Application Publication No. 2001/0137843
U.S. Patent Application Publication No. 2011/0257429
U.S. Patent Application Publication No. 2011/0300589
U.S. Patent Application Publication No. 2012/0142945
U.S. Patent Application Publication No. 2012/0238722
U.S. Patent Application Publication No. 2013/0018206
U.S. Patent Application Publication No. 2013/0096343
U.S. Patent Application Publication No. 2013/0081947
International Patent Application Publication No. WO2005/051885
International Patent Application Publication No. WO2009/030395
International Patent Application Publication No. WO2009/040095
International Patent Application Publication No. WO2009/082050
International Patent Application Publication No. WO2010/022960
International Patent Application Publication No. WO2010/022966
International Patent Application Publication No. WO2011/064151
International Patent Application Publication No. WO2011/069007

International Patent Application Publication No. WO2011/123269

International Patent Application Publication No. WO2011/160760

International Patent Application Publication No. WO2013/012590

Benedict, D. J., Parulekar, S. J. and Tsai, S-P. (2006) Pervaporation-assisted esterification and succinic acids with downstream ester recovery. J. Mem. Sci. 281: 435-445.

Budarin, V., Luque, R., Macquarrie, D. J. and Clark, J. H. (2007) Towards a bio-based industry: Benign catalytic esterification of succinic acid in the presence of the water. Chem. Euro. J. 13: 6914-6919.

Cheng, Ke-ke., Zhao, X-B., Zeng, J., Wu, R-C., Xu, Y-Z., Liu, D-H., Zhang, J-A. (2012) Downstream processing of biotechnological produced succinic acid. 95: 841-850.

Davis, S. 1, 4-Butanediol, CEH Market Research Report, October 2010.

Davison, B. H., Nghiem, N. P. and Richardson, G. L. (2004) Succinic acid adsorption from fermentation broth and regeneration. App. Biochem. Biotechnol. 113-116: 653-669.

Delhomme, C., Weuster-Botz, D., Kuhn, F. E. (2009) Succinic acid from renewable resources ad a C4 building-block chemical - A review of the catalytic possibilities in aqueous media. Green Chem. 11: 13-26.

Delhomme, C. (2011) Process integration of fermentation and catalysts for the production of succinic acid derivatives. Dissertation submitted to degree of Doctor of Philosophy in Engineering, Technische Universitat Munchen, Germany.

Delhomme, C., Goh, S. L. M., Kuhn, F. E. and Weuster-Botz, D. (2012) Esterification of bio-based succinic acid in biphasic systems: Comparison of chemical and biological catalysts. J. Mol. Cata. Enz. 80: 39-47.

Filachione, E. M., Costello, E. J. and C. H. Fisher. (1951) Preparation of esters by reaction of ammonium salts with alcohols. J. Amer. Chem. Society. 13(11), 5265-5267.

Filachione, E. M. and Costello, E. J. (1952) Lactic esters by reaction of ammonium lactate with alcohols. Ind. & Eng. Chem., 44, 2189-2191.

Huh, Y. S., Jun, Y-S., Hong, Y. K., Song, H., Lee, S. Y., Hong, W. H. (2006) Effective purification of succinic acid from fermentation broth produced by Mannheimia succiniciproducens. Proc. Biochem. 41: 1461-1465.

Jafar, J. J., Budd, P. M., Hughes, R. (2002) Enhancement of esterification reaction yield using zeolite Avapour permeation membrane. J. Mem. Sci. 199: 117-123.

Jantama, K., Haupt, M. J., Svoronos, S. A., Zhang, X., Moore, J. C., Shanmugham, K. T., Ingram, L. O. (2008) Combining metabolic engineering and metabolic evolution to develop nonrecombinant strains of E. coli C that produce succinate and malate. Biotech Bioeng 99: 1140-1153.

Jantama, K., Zhang, X., Moore, J. C., Shanmugham, K. T., Svoronos, S. A., Ingram, L. O. (2008) Eliminating side products and increasing succinate yields in engineered strains of Escherichia coli C. Biotech. Bioeng. 101: 881-893.

Jun, Y-S., Huh, Y. S., Hong, W. H. and Hong, Y. K. (2005) Kinetics of the extraction of succinic acid with tri-octylamine in 1-octanol solution. Biotechnol. Prog. 21: 1673-1679.

Jun, Y-S., Lee, E. Z., Huh, Y. S., Hong, Y. K. (2007) Kinetic study for the extraction of succinic acid with ZTOA in fermentation broth; effects of pH, salt and contaminated acid. Biochem. Eng. J. 36: 8-13.

Kurzrock, T. and Weuster-Botz, D. (2009) Recovery of succinic acid from fermentation broth. Biotechnol. Lett. DOI 10.1007/s10529-009-0163-6. Published online: 08 November 2009.

Kurzrock, T. and Weuster-Botz, D. (2011) New reactive extraction systems for separation of bio-succinic acid. Bioprocess Biosyst. Eng. 34: 779-787.

Li, Q., Wang, D., Wu, Y., Li, W., Zhang, Y., Xing, J. and Su, Z. (2010) One Step recovery of succinic acid from fermentation broths by crystallization. Sep. Purif. Technol. 72: 294-300.

Li, Q., Li, W-1., Wang, D., Lilu, B-b., Tang, H., Yang, M-h., Liu, Q-f., Xing, J. M. and Su, Z-g. (2010) pH Neutralization while succinic acid adsorption onto anion-exchange resins. Appl Biochem Biotechnol. 160:438-445.

Lin, C.S.K., Luque, R., Clark, J. H., Webb, C. and Du, C. (2011) Wheat-based biorefining strategy for fermentative production and chemical transformations of succinic acid. Biofuels, Bioprod. Bioref. 6: 88-104.

Londono, A. O. (2010) Separation of succinic acid from fermentation broths and esterification by a reactive distillation method. A dissertation submitted for the degree of Doctor of Philosophy in Chemical Engineering, Michigan State University, USA.

Lopez-Garzon, C. S., Ottens, M., van der Wielen, A. M. and Straathof, A. J. J. (2012) Direct downstream catalysis: From succinate to its diethyl ester without intermediate acidification. Chem.Eng. J. 200-202: 637-644.

Luque, R., Lin, C. S. K., Du, C., Macquarrie, D. J., Koutinas, A., Wang, R., Webb, C. and Clark, J. H. (2009) Chemical transformations of succinic acid recovered from fermentation broths by a novel direct vacuum distillation-crystallization method. Green Chem. 11: 193-200.

Minh, D. P., Besson, M., Pinel, C., Fuertes, P. and Petitjean, C. (2010) Aqueous-phase hydrogenation of biomass-based succinic acid to 1,4-butanediol over supported bimetallic catalysts. Top. Catal. 53: 1270-1273.

Orjuela, A., Kolah, A., Lilra, C. T. and Miller, D. J. (2011) Mixed succinic acid/acetic acid esterification with ethanol by reactive distillation. Ind. Eng. Chem. Res. 50: 9209-9220.

Orjuela, A., Yanez, A., Peereboom, L., Lira, C. T. and Miller, D. J. (2011) A novel process for recovery of fermentation-derived succinic acid. Sep. Purif. Tech. 83: 31-37.

Orjuela, A., Yanez, A. J., Santhanakrishnan, A., Lira, C. T. and Miller, D. J. (2012) Kinetics of mixed succinic acid/acetic acid esterification with Amberlyst 70 ion exchange resin as catalyst. Chem. Eng. J. 188: 98-107.

Orjuela, A., Kolah, A., Hong, X., Lira, C. T. and Miller, D.J. (2012) Diehtyl succinate synthesis by reactive distillation. Sep. Purif. Tech. 88: 151-162.

Orjuela, A., Orjuela, A., Lira, C. T. and Miller, D. J. (2013) A novel process for recovery of fermentation-derived succinic acid: Process design and economic analysis. Bioreso. Tech. 139: 235-241.

Rahman, M. B. A., Jarmi, N. I., Chaibakhsh, N. and Basri, M. (2011) Modeling and optimization of lipase-catalysed production of succinic acid ester using central composite design analysis. J. Ind. Microbiol. Biotechnol. 38: 229-234.

Song, H., Huh, Y. S., Lee, S. Y., Hong, W. H. and Hong, Y. K. (2007) Recovery of succinic acid produced by fermentation of a metabolically engineered Mannheimia succiniciproducens strain. J. Biotechnol. 132: 445-452.

Varadarajan, S. and Miller, D. J. (1999) Catalytic upgrading of fermentation-derived organic acids. Biotechnol. Prog. 15: 845-854

Wang, C., Li, Q., Tang, H., Zhou, W., Yan, D., xing, J. and Wan, Y. (2012) Clarification of succinic acid fermentation broth by ultrafiltration in succinic acid bio-refinery. J. Chem. Technol. Biotechnol. 88: 444-448.

Zhang, X., Jantama, K., Moore, J. C., Shanmugam, K. T., Ingram, L. O. (2009) Metabolic evolution of energy-conserving pathways for succinate production in Escherichia coli. Proc. Natl. Acad. Sci. U.S. A. 106: 20180-20185.

## Claims

1. A process for preparing succinate ester comprising the steps of:

   (a) fermentation of carbohydrate substrates using a biocatalyst to produce succinate salt;
   (b) clarification of fermentation broth to remove biocatalyst and insolubles;
   (c) acidification of fermentation broth from step (b) with phosphoric acid;
   (d) controlled crystallization of succinic acid in the acidified fermentation broth from step (c);
   (e) separating crystalline succinic acid from mother liquor;
   (f) dissolving said crystalline succinic acid crystal from step (e) in an alcohol;
   (g) esterification of said succinic acid dissolved in alcohol in step (f); and
   (h) recovering succinic acid ester through distillation.

2. A process for preparing succinate ester as in claim 1 wherein said alcohol is methanol.

3. A process for preparing succinate ester as in claim 1 wherein said alcohol is ethanol.

4. A process for preparing succinate ester as in claim 1 further comprising the steps of:

   (a) transferring said mother liquor to an evaporator to produce a concentrated mother liquor;
   (b) transferring said concentrated mother liquor to a crystallizer to produce a slurry;
   (c) centrifuging said slurry from step (b) to obtain a precipitate and washing said precipitate in an alcohol to recover ammonium sulfate as crystals and an alcohol fraction comprising succinic acid;
   (d) esterifying said succinic acid in alcohol in step (c); and
   (e) distilling said succinic acid ester from step (d).

5. A process for preparing succinate ester as in claim 4 wherein said alcohol is ethanol.

6. A process for preparing succinate ester as in claim 4 wherein said alcohol is methanol.

7. A process for preparing succinic acid comprising the steps of:

   (a) fermentation of carbohydrate substrates using a biocatalyst to produce succinate salt;
   (b) clarification of fermentation broth to remove biocatalyst and insolubles;
   (c) acidification of fermentation broth from step (b) with phosphoric acid;
   (d) controlled crystallization of succinic acid in the acidified fermentation broth from step (c);
   (e) separating crystalline succinic acid from mother liquor;
   (f) dissolving said crystalline succinic acid crystal from step (e) in an alcohol;
   (g) transferring said mother liquor to an evaporator to produce a concentrated mother liquor;

(h) transferring said concentrated mother liquor to a crystallizer to produce a slurry;

(i) centrifuging said slurry from step (h) to obtain a precipitate and washing said precipitate in an alcohol to recover ammonium sulfate as crystals and an alcohol fraction comprising succinic acid;

(j) combining said alcohol fraction comprising succinic acid in step (i) with said succinic acid dissolved in alcohol in step (f);

(k) esterifying said succinic acid in alcohol in step (j);

(l) distilling said succinic acid ester from step (k); and

(m) hydrolyzing succinic acid ester from step (l) to produce succinic acid.

8. A process for preparing succinate ester comprising the steps of:

   (a) fermentation of carbohydrate substrates using a biocatalyst to produce succinate salt;
   (b) clarification of fermentation broth to remove biocatalyst and insolubles;
   (c) mixing said fermentation broth from step (b) with an alcohol to salt out succinate salt;
   (d) filtering said salted out succinate salt in step (c);
   (e) redissolving said salted out succinate salt in step (d) in methanol in the presence of sulfuric acid causing precipitation of sulfate;
   (f) filtering said sulfate from step (e) and recovering filtrate comprising succinic acid and dimethyl succinate;
   (g) esterifying said succinic acid in said filtrate in step (f); and
   (h) recovering succinic acid ester through distillation.

9. A process for preparing succinate ester as in claim 8 wherein said alcohol used to salt out succinate salt is methanol.

10. A process for preparing succinate ester as in claim 8 wherein sulfur content of said succinate ester is below 50 ppm level.

11. A process for preparing succinate ester as in claim 8 wherein sulfur content of said succinate ester is below 10 ppm level.

**Patentansprüche**

1. Verfahren zur Herstellung von Succinatester, umfassend die Schritte:

   (a) Fermentieren von Kohlenhydratsubstraten unter Verwendung eines Biokatalysators, um ein Succinatsalz herzustellen;
   (b) Klären der Fermentationsbrühe, um den Biokatalysator und unlösliche Bestandteile zu entfernen;
   (c) Ansäuern der Fermentationsbrühe aus Schritt (b) mit Phosphorsäure;
   (d) kontrolliertes Kristallisieren von Bernsteinsäure in der angesäuerten Fermentationsbrühe aus Schritt (c);
   (e) Abtrennen der kristallinen Bernsteinsäure von der Mutterlauge;
   (f) Auflösen des kristallinen Bernsteinsäurekristalls aus Schritt (e) in einem Alkohol;
   (g) Verestern der in Alkohol aufgelösten Bernsteinsäure aus Schritt (f); und
   (h) Rückgewinnen des Bernsteinsäureesters durch Destillation.

2. Verfahren zur Herstellung von Succinatester nach Anspruch 1, wobei der Alkohol Methanol ist.

3. Verfahren zur Herstellung von Succinatester nach Anspruch 1, wobei der Alkohol Ethanol ist.

4. Verfahren zur Herstellung von Succinatester nach Anspruch 1, weiter umfassend die Schritte:

   (a) Überführen der Mutterlauge zu einem Verdampfer, um eine konzentrierte Mutterlauge herzustellen;
   (b) Überführen der konzentrierten Mutterlauge zu einem Kristallisator, um eine Aufschlämmung herzustellen;
   (c) Zentrifugieren der Aufschlämmung aus Schritt (b), um einen Niederschlag zu erhalten, und Waschen des Niederschlags in einem Alkohol, um Ammoniumsulfat als Kristalle und eine Bernsteinsäure umfassende Alkoholfraktion rückzugewinnen;
   (d) Verestern der Bernsteinsäure in Alkohol aus Schritt (c); und
   (e) Destillieren des Bernsteinsäureesters aus Schritt (d).

**5.** Verfahren zur Herstellung von Succinatester nach Anspruch 4, wobei der Alkohol Ethanol ist.

**6.** Verfahren zur Herstellung von Succinatester nach Anspruch 4, wobei der Alkohol Methanol ist.

**7.** Verfahren zur Herstellung von Bernsteinsäure, umfassend die Schritte:

(a) Fermentieren von Kohlenhydratsubstraten unter Verwendung eines Biokatalysators, um ein Succinatsalz herzustellen;
(b) Klären der Fermentationsbrühe, um den Biokatalysator und unlösliche Bestandteile zu entfernen;
(c) Ansäuern der Fermentationsbrühe aus Schritt (b) mit Phosphorsäure;
(d) kontrolliertes Kristallisieren von Bernsteinsäure in der angesäuerten Fermentationsbrühe aus Schritt (c);
(e) Abtrennen der kristallinen Bernsteinsäure von der Mutterlauge;
(f) Auflösen des kristallinen Bernsteinsäurekristalls aus Schritt (e) in einem Alkohol;
(g) Überführen der Mutterlauge zu einem Verdampfer, um eine konzentrierte Mutterlauge herzustellen;
(h) Überführen der konzentrierten Mutterlauge zu einem Kristallisator, um eine Aufschlämmung herzustellen;
(i) Zentrifugieren der Aufschlämmung aus Schritt (h), um einen Niederschlag zu erhalten, und Waschen des Niederschlags in einem Alkohol, um Ammoniumsulfat als Kristalle und eine Bernsteinsäure umfassende Alkoholfraktion rückzugewinnen;
(j) Vereinigen der Bernsteinsäure umfassenden Alkoholfraktion aus Schritt (i) mit der in Alkohol aufgelösten Bernsteinsäure aus Schritt (f);
(k) Verestern der Bernsteinsäure in Alkohol aus Schritt (j);
(l) Destillieren des Bernsteinsäureesters aus Schritt (k); und
(m) Hydrolysieren des Bernsteinsäureesters aus Schritt (l), um Bernsteinsäure herzustellen.

**8.** Verfahren zur Herstellung von Succinatester, umfassend die Schritte:

(a) Fermentieren von Kohlenhydratsubstraten unter Verwendung eines Biokatalysators, um ein Succinatsalz herzustellen;
(b) Klären der Fermentationsbrühe, um den Biokatalysator und unlösliche Bestandteile zu entfernen;
(c) Mischen der Fermentationsbrühe aus Schritt (b) mit einem Alkohol, um das Succinatsalz auszusalzen;
(d) Filtrieren des ausgesalzenen Succinatsalzes aus Schritt (c);
(e) erneutes Auflösen des ausgesalzenen Succinatsalzes aus Schritt (d) in Methanol in der Gegenwart von Schwefelsäure, was den Niederschlag von Sulfat herbeiführt;
(f) Filtrieren des Sulfats aus Schritt (e) und Rückgewinnen des Bernsteinsäure und Dimethylsuccinat umfassenden Filtrats;
(g) Verestern der Bernsteinsäure in dem Filtrat aus Schritt (f); und
(h) Rückgewinnen des Bernsteinsäureesters durch Destillation.

**9.** Verfahren zur Herstellung von Succinatester nach Anspruch 8, wobei der zum Aussalzen des Succinatsalzes verwendete Alkohol Methanol ist.

**10.** Verfahren zur Herstellung von Succinatester nach Anspruch 8, wobei der Schwefelgehalt des Succinatesters unterhalb eines Werts von 50 ppm liegt.

**11.** Verfahren zur Herstellung von Succinatester nach Anspruch 8, wobei der Schwefelgehalt des Succinatesters unterhalb eines Werts von 10 ppm liegt.

**Revendications**

**1.** Procédé de préparation d'ester de succinate comprenant les étapes de :

(a) fermentation de substrats glucidiques en utilisant un biocatalyseur pour produire un sel succinate ;
(b) clarification d'un bouillon de fermentation pour éliminer le biocatalyseur et les substances insolubles ;
(c) acidification du bouillon de fermentation de l'étape (b) avec de l'acide phosphorique ;
(d) cristallisation contrôlée de l'acide succinique dans le bouillon de fermentation acidifié de l'étape (c) ;
(e) séparation de l'acide succinique cristallin de la liqueur mère ;
(f) dissolution dudit cristal d'acide succinique cristallin de l'étape (e) dans un alcool ;

(g) estérification dudit acide succinique dissous dans l'alcool à l'étape (f) ; et

(h) récupération d'ester d'acide succinique par distillation.

**2.** Procédé de préparation d'ester de succinate selon la revendication 1 dans lequel ledit alcool est du méthanol.

**3.** Procédé de préparation d'ester de succinate selon la revendication 1 dans lequel ledit alcool est de l'éthanol.

**4.** Procédé de préparation d'ester de succinate selon la revendication 1 comprenant en outre les étapes de :

(a) transfert de ladite liqueur mère à un évaporateur pour produire une liqueur mère concentrée ;

(b) transfert de ladite liqueur mère concentrée à un cristalliseur pour produire une suspension ;

(c) centrifugation de ladite suspension de l'étape (b) pour obtenir un précipité et lavage dudit précipité dans un alcool pour récupérer du sulfate d'ammonium sous forme de cristaux et une fraction alcoolique comprenant de l'acide succinique ;

(d) estérification dudit acide succinique en alcool de l'étape (c) ; et

(e) distillation dudit ester d'acide succinique de l'étape (d).

**5.** Procédé de préparation d'ester de succinate selon la revendication 4 dans lequel ledit alcool est de l'éthanol.

**6.** Procédé de préparation d'ester de succinate selon la revendication 4 dans lequel ledit alcool est du méthanol.

**7.** Procédé de préparation d'acide succinique comprenant les étapes de :

(a) fermentation de substrats glucidiques en utilisant un biocatalyseur pour produire un sel succinate ;

(b) clarification d'un bouillon de fermentation pour éliminer le biocatalyseur et les substances insolubles ;

(c) acidification du bouillon de fermentation de l'étape (b) avec de l'acide phosphorique ;

(d) cristallisation contrôlée de l'acide succinique dans le bouillon de fermentation acidifié de l'étape (c) ;

(e) séparation de l'acide succinique cristallin de la liqueur mère ;

(f) dissolution dudit cristal d'acide succinique cristallin de l'étape (e) dans un alcool ;

(g) transfert de ladite liqueur mère à un évaporateur pour produire une liqueur mère concentré ;

(h) transfert de ladite liqueur mère concentrée à un cristalliseur pour produire une suspension ;

(i) centrifugation de ladite suspension de l'étape (h) pour obtenir un précipité et lavage dudit précipité dans un alcool pour récupérer du sulfate d'ammonium sous forme de cristaux et une fraction alcoolique comprenant de l'acide succinique ;

(j) combinaison de ladite fraction alcoolique comprenant de l'acide succinique de l'étape (i) avec ledit acide succinique dissous dans l'alcool de l'étape (f) ;

(k) estérification dudit acide succinique en alcool de l'étape (j) ;

(l) distillation dudit ester d'acide succinique de l'étape (k) ; et

(m) hydrolyse de l'ester d'acide succinique de l'étape (l) pour produire de l'acide succinique.

**8.** Procédé de préparation d'ester de succinate comprenant les étapes de :

(a) fermentation de substrats glucidiques en utilisant un biocatalyseur pour produire un sel succinate ;

(b) clarification d'un bouillon de fermentation pour éliminer le biocatalyseur et les substances insolubles ;

(c) mélange dudit bouillon de fermentation de l'étape (b) avec un alcool pour extraire du sel succinate ;

(d) filtrage dudit sel succinate extrait à l'étape (c) ;

(e) redissolution dudit sel succinate extrait à l'étape (d) dans du méthanol en présence d'acide sulfurique entraînant une précipitation de sulfate ;

(f) filtrage dudit sulfate de l'étape (e) et récupération du filtrat comprenant de l'acide succinique et du succinate de diméthyle ;

(g) estérification dudit acide succinique dans ledit filtrat de l'étape (f) ; et

(h) récupération d'ester d'acide succinique par distillation.

**9.** Procédé de préparation d'ester de succinate selon la revendication 8 dans lequel ledit alcool utilisé pour extraire le sel succinate est du méthanol.

**10.** Procédé de préparation d'ester de succinate selon la revendication 8 dans lequel une teneur en soufre dudit ester de succinate est inférieure à un niveau de 50 ppm.

11. Procédé de préparation d'ester de succinate selon la revendication 8 dans lequel une teneur en soufre dudit ester de succinate est inférieure à un niveau de 10 ppm.

# Figure 1

# Figure 2

Flowsheet 1

96% $H_2SO_4$

**Ammonium succinate broth**

Acidification
pH < 4.5

Concentration

Condensate with
Acetic Acid

Cooling
Crystallization

Filtration

Crude Succinic
Acid Crystal

Mother liquor

Flowsheet 2a

**Ammonium succinate broth**

Evaporation

Condensate with
Ammonia

Cooling

96% $H_2SO_4$

Precipitation
pH < 4.5

Filtration

Crude Succinic
Acid Crystal

Mother liquor

Flowsheet 2b

**Ammonium Succinate broth**

Evaporation

Condensate with
Ammonia

96% $H_2SO_4$

Precipitation

Heating

Recrystallization

Filtration

Crude Succinic
Acid Crystal

Mother liquor

# Figure 3

**Normal Process:**

Maintain all AMS and SAC soluble, and then slowly crystallize out SAC.

H2SO4

Ammonium Succinate Broth ~pH 6.5

AMS + SAC ~pH 2.2-2.4

Crystallization

**Two pH Stage Crystallization:**

Crystallize out a portion of SAC before adding more H2SO4.

H2SO4

H2SO4

Ammonium Succinate Broth ~pH 6.5

AMS + SAC ~pH 2.8-3.0

1st Crystallization

~pH 2.2-2.4
2nd Crystallization

EP 3 077 525 B1

34

**Figure 4A**

# Figure 4B

EP 3 077 525 B1

# Figure 5

# Figure 6A

EP 3 077 525 B1

# Figure 6B

Figure 7

## Figure 8

# Figure 9

**Figure 10**

Figure 11

Figure 12

AMSAC: 8.8 g

H2SO4: 11 g
(Added 2x of
the theoretical
equivalents
required)

MeOH: 30-37 g

Salt Splitting

Liquid stream:
$0.8 - 1.1$ g AMS
$6 - 6.5$ g DMS
$15 - 24$ g MeOH

Solids:
$7 - 7.5$ g AMS
3% moisture
$2 - 3$ g unknown

Figure 13A

**Figure 13B**

# FIGURE 13C

# FIGURE 14

**FIGURE 15A**

**FIGURE 15B**

**FIGURE 15C**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5168055 A
- US 5034105 A
- US 5143834 A
- WO 2011160760 A
- US 20130096343 A
- WO 2011123269 A
- US 5958744 A
- US 6265190 B
- WO 2008115958 A
- WO 2010115067 A
- US 2565487 A
- US 2722541 A
- US 2766273 A
- US 4100189 A
- US 4405717 A
- US 4444881 A
- US 4645855 A
- US 4885247 A
- US 5068418 A
- US 5132456 A
- US 5210296 A
- US 5323639 A
- US 5352825 A
- US 5405992 A
- US 5449824 A
- US 5453365 A
- US 5502240 A
- US 5510526 A
- US 5641406 A
- US 5766439 A
- US 5773653 A
- US 5780276 A
- US 5780678 A
- US 5786185 A
- US 5892109 A
- US 5986133 A
- US 6043392 A
- US 6087532 A
- US 6160173 A
- US 6187951 B
- US 6229046 B
- US 6280985 B
- US 6291708 B
- US 6319382 B
- US 6320077 B
- US 6342626 B
- US 6384276 B
- US 6403825 B
- US 64520151 B

- US 6472559 B
- US 6476239 B
- US 6478965 B
- US 6509179 B
- US 6518454 B
- US 6534079 B
- US 6583310 B
- US 6660505 B
- US 6641734 B
- US 6664413 B
- US 6670505 B
- US 6772213 B
- US 6803217 B
- US 6872314 B
- US 6926810 B
- US 6942803 B
- US 7019170 B
- US 7026145 B
- US 7144977 B
- US 7186856 B
- US 7238837 B
- US 7297809 B
- US 7439392 B
- US 7498450 B
- US 7528277 B
- US 7601865 B
- US 7632479 B
- US 7652167 B
- US 7696375 B
- US 7709689 B
- US 7754916 B
- US 7820859 B
- US 7915447 B
- US 8084626 B
- US 8193375 B
- US 8203021 B
- US 8241482 B
- US 8293935 B
- US 8338145 B
- US 8410306 B
- US 20040210087 A
- US 20060252956 A
- US 20060276674 A
- US 20060041165 A
- US 20070014895 A
- US 20070096481 A
- US 20070161816 A
- US 20070167649 A
- US 20080287714 A

- US 2008042958 A
- US 20090076297 A
- US 20090104322 A
- US 20090234160 A
- US 20100297715 A
- US 20010137843 A
- US 20110257429 A
- US 20110300589 A
- US 20120142945 A
- US 20120238722 A
- US 20130018206 A

- US 20130081947 A
- WO 2005051885 A
- WO 2009030395 A
- WO 2009040095 A
- WO 2009082050 A
- WO 2010022960 A
- WO 2010022966 A
- WO 2011064151 A
- WO 2011069007 A
- WO 2013012590 A

**Non-patent literature cited in the description**

- **BENEDICT, D. J. ; PARULEKAR, S. J. ; TSAI, S-P.** Pervaporation-assisted esterification and succinic acids with downstream ester recovery. *J. Mem. Sci.,* 2006, vol. 281, 435-445
- **BUDARIN, V. ; LUQUE, R. ; MACQUARRIE, D. J. ; CLARK, J. H.** Towards a bio-based industry: Benign catalytic esterification of succinic acid in the presence of the water. *Chem. Euro. J.,* 2007, vol. 13, 6914-6919
- **CHENG, KE-KE. ; ZHAO, X-B. ; ZENG, J. ; WU, R-C. ; XU, Y-Z. ; LIU, D-H. ; ZHANG, J-A.** *Downstream processing of biotechnological produced succinic acid,* 2012, vol. 95, 841-850
- **DAVIS, S.** *1, 4-Butanediol, CEH Market Research Report,* October 2010
- **DAVISON, B. H. ; NGHIEM, N. P. ; RICHARDSON, G. L.** Succinic acid adsorption from fermentation broth and regeneration. *App. Biochem. Biotechnol.,* 2004, vol. 113-116, 653-669
- **DELHOMME, C. ; WEUSTER-BOTZ, D. ; KUHN, F. E.** Succinic acid from renewable resources ad a C4 building-block chemical - A review of the catalytic possibilities in aqueous media. *Green Chem.,* 2009, vol. 11, 13-26
- Process integration of fermentation and catalysts for the production of succinic acid derivatives. **DELHOMME, C.** Dissertation submitted to degree of Doctor of Philosophy in Engineering. Technische Universitat Munchen, 2011
- **DELHOMME, C. ; GOH, S. L. M. ; KUHN, F. E. ; WEUSTER-BOTZ, D.** Esterification of bio-based succinic acid in biphasic systems: Comparison of chemical and biological catalysts. *J. Mol. Cata. Enz.,* 2012, vol. 80, 39-47
- **FILACHIONE, E. M. ; COSTELLO, E. J. ; C. H. FISHER.** Preparation of esters by reaction of ammonium salts with alcohols. *J. Amer. Chem. Society.,* 1951, vol. 13 (11), 5265-5267
- **FILACHIONE, E. M. ; COSTELLO, E. J.** Lactic esters by reaction of ammonium lactate with alcohols. *Ind. & Eng. Chem.,* 1952, vol. 44, 2189-2191

- **HUH, Y. S. ; JUN, Y-S. ; HONG, Y. K. ; SONG, H. ; LEE, S. Y. ; HONG, W. H.** Effective purification of succinic acid from fermentation broth produced by Mannheimia succiniciproducens. *Proc. Biochem.,* 2006, vol. 41, 1461-1465
- **JAFAR, J. J. ; BUDD, P. M. ; HUGHES, R.** Enhancement of esterification reaction yield using zeolite Avapour permeation membrane. *J. Mem. Sci.,* 2002, vol. 199, 117-123
- **JANTAMA, K. ; HAUPT, M. J. ; SVORONOS, S. A. ; ZHANG, X. ; MOORE, J. C. ; SHANMUGHAM, K. T. ; INGRAM, L. O.** Combining metabolic engineering and metabolic evolution to develop nonrecombinant strains of E. coli C that produce succinate and malate. *Biotech Bioeng,* 2008, vol. 99, 1140-1153
- **JANTAMA, K. ; ZHANG, X. ; MOORE, J. C. ; SHANMUGHAM, K. T. ; SVORONOS, S. A. ; INGRAM, L. O.** Eliminating side products and increasing succinate yields in engineered strains of Escherichia coli C. *Biotech. Bioeng.,* 2008, vol. 101, 881-893
- **JUN, Y-S. ; HUH, Y. S. ; HONG, W. H. ; HONG, Y. K.** Kinetics of the extraction of succinic acid with tri-octylamine in 1-octanol solution. *Biotechnol. Prog.,* 2005, vol. 21, 1673-1679
- **JUN, Y-S. ; LEE, E. Z. ; HUH, Y. S. ; HONG, Y. K.** Kinetic study for the extraction of succinic acid with ZTOA in fermentation broth; effects of pH, salt and contaminated acid. *Biochem. Eng. J.,* 2007, vol. 36, 8-13
- **KURZROCK, T. ; WEUSTER-BOTZ, D.** Recovery of succinic acid from fermentation broth. *Biotechnol. Lett.,* 08 November 2009
- **KURZROCK, T. ; WEUSTER-BOTZ, D.** New reactive extraction systems for separation of bio-succinic acid. *Bioprocess Biosyst. Eng.,* 2011, vol. 34, 779-787
- **LI, Q. ; WANG, D. ; WU, Y. ; LI, W. ; ZHANG, Y. ; XING, J. ; SU, Z.** One Step recovery of succinic acid from fermentation broths by crystallization. *Sep. Purif. Technol.,* 2010, vol. 72, 294-300

- **LI, Q. ; LI, W-1. ; WANG, D. ; LILU, B-B. ; TANG, H. ; YANG, M-H. ; LIU, Q-F. ; XING, J. M. ; SU, Z-G.** pH Neutralization while succinic acid adsorption onto anion-exchange resins. *Appl Biochem Biotechnol.,* 2010, vol. 160, 438-445
- **LIN, C.S.K. ; LUQUE, R. ; CLARK, J. H. ; WEBB, C. ; DU, C.** Wheat-based biorefining strategy for fermentative production and chemical transformations of succinic acid. *Biofuels, Bioprod. Bioref.,* 2011, vol. 6, 88-104
- Separation of succinic acid from fermentation broths and esterification by a reactive distillation method. **LONDONO, A. O.** A dissertation submitted for the degree of Doctor of Philosophy in Chemical Engineering. Michigan State University, 2010
- **LOPEZ-GARZON, C. S. ; OTTENS, M. ; VAN DER WIELEN, A. M. ; STRAATHOF, A. J. J.** Direct downstream catalysis: From succinate to its diethyl ester without intermediate acidification. *Chem.Eng. J.,* 2012, vol. 200-202, 637-644
- **LUQUE, R. ; LIN, C. S. K. ; DU, C. ; MACQUARRIE, D. J. ; KOUTINAS, A. ; WANG, R. ; WEBB, C. ; CLARK, J. H.** Chemical transformations of succinic acid recovered from fermentation broths by a novel direct vacuum distillation-crystallization method. *Green Chem.,* 2009, vol. 11, 193-200
- **MINH, D. P. ; BESSON, M. ; PINEL, C. ; FUERTES, P. ; PETITJEAN, C.** Aqueous-phase hydrogenation of biomass-based succinic acid to 1,4-butanediol over supported bimetallic catalysts. *Top. Catal.,* 2010, vol. 53, 1270-1273
- **ORJUELA, A. ; KOLAH, A. ; LILRA, C. T. ; MILLER, D. J.** Mixed succinic acid/acetic acid esterification with ethanol by reactive distillation. *Ind. Eng. Chem. Res.,* 2011, vol. 50, 9209-9220
- **ORJUELA, A. ; YANEZ, A. ; PEEREBOOM, L. ; LIRA, C. T. ; MILLER, D. J.** A novel process for recovery of fermentation-derived succinic acid. *Sep. Purif. Tech.,* 2011, vol. 83, 31-37
- **ORJUELA, A. ; YANEZ, A. J. ; SANTHANA-KRISHNAN, A. ; LIRA, C. T. ; MILLER, D. J.** Kinetics of mixed succinic acid/acetic acid esterification with Amberlyst 70 ion exchange resin as catalyst. *Chem. Eng. J.,* 2012, vol. 188, 98-107
- **ORJUELA, A. ; KOLAH, A. ; HONG, X. ; LIRA, C. T. ; MILLER, D.J.** Diehtyl succinate synthesis by reactive distillation. *Sep. Purif. Tech.,* 2012, vol. 88, 151-162
- **ORJUELA, A. ; ORJUELA, A. ; LIRA, C. T. ; MILLER, D. J.** A novel process for recovery of fermentation-derived succinic acid: Process design and economic analysis. *Bioreso. Tech.,* 2013, vol. 139, 235-241
- **RAHMAN, M. B. A. ; JARMI, N. I. ; CHAIBAKHSH, N. ; BASRI, M.** Modeling and optimization of lipase-catalysed production of succinic acid ester using central composite design analysis. *J. Ind. Microbiol. Biotechnol.,* 2011, vol. 38, 229-234
- **SONG, H. ; HUH, Y. S. ; LEE, S. Y. ; HONG, W. H. ; HONG, Y. K.** Recovery of succinic acid produced by fermentation of a metabolically engineered Mannheimia succiniciproducens strain. *J. Biotechnol.,* 2007, vol. 132, 445-452
- **VARADARAJAN, S. ; MILLER, D. J.** Catalytic upgrading of fermentation-derived organic acids. *Biotechnol. Prog.,* 1999, vol. 15, 845-854
- **WANG, C. ; LI, Q. ; TANG, H. ; ZHOU, W. ; YAN, D. ; XING, J. ; WAN, Y.** Clarification of succinic acid fermentation broth by ultrafiltration in succinic acid bio-refinery. *J. Chem. Technol. Biotechnol.,* 2012, vol. 88, 444-448
- **ZHANG, X. ; JANTAMA, K. ; MOORE, J. C. ; SHANMUGAM, K. T. ; INGRAM, L. O.** Metabolic evolution of energy-conserving pathways for succinate production in Escherichia coli. *Proc. Natl. Acad. Sci. U.S. A.,* 2009, vol. 106, 20180-20185